(19) Europäisches Patentamt
European Patent Office
Office européen des brevets

(11) EP 1 202 997 B1

(12) **EUROPÄISCHE PATENTSCHRIFT**

(45) Veröffentlichungstag und Bekanntmachung des Hinweises auf die Patenterteilung:
**17.09.2003 Patentblatt 2003/38**

(21) Anmeldenummer: **00951376.3**

(22) Anmeldetag: **12.07.2000**

(51) Int Cl.[7]: **C07F 7/18**, C07F 7/21, C08L 83/10, A61K 6/093

(86) Internationale Anmeldenummer:
**PCT/EP00/06639**

(87) Internationale Veröffentlichungsnummer:
**WO 01/007444 (01.02.2001 Gazette 2001/05)**

(54) **HYDROLYSIERBARE UND POLYMERISIERBARE SILANE MIT GERINGER VISKOSITÄT UND DEREN VERWENDUNG**

HYDROLYZABLE SILANES AND POLYMERIZABLE SILANES WITH LOW VISCOSITY AND USE THEREOF

SILANES HYDROLYSABLES ET POLYMERISABLES A FAIBLE VISCOSITE ET LEUR UTILISATION

(84) Benannte Vertragsstaaten:
**AT BE CH CY DE DK ES FI FR GB GR IE IT LI LU MC NL PT SE**

(30) Priorität: **22.07.1999 DE 19934407**

(43) Veröffentlichungstag der Anmeldung:
**08.05.2002 Patentblatt 2002/19**

(73) Patentinhaber: **3M Espe AG**
**82229 Seefeld (DE)**

(72) Erfinder:
- **BISSINGER, Peter**
**D-86911 Diessen (DE)**
- **GASSER, Oswald**
**D-82229 Seefeld (DE)**
- **GUGGENBERGER, Rainer**
**D-82211 Herrsching (DE)**
- **SOGLOWEK, Wolfgang**
**D-86911 Diessen-Obermühlhausen (DE)**

(74) Vertreter: **Freiherr von Wittgenstein, Arved, Dr.**
**Patentanwälte Abitz & Partner**
**Postfach 86 01 09**
**81628 München (DE)**

(56) Entgegenhaltungen:
**EP-A- 0 963 751**
**WO-A-94/06807**
**WO-A-98/22521**
**DE-A- 19 860 361**

- **ISHIKAWA, MITSUO ET AL: "Silicon-carbon unsaturated compounds. 33. Regiochemistry in the photochemical formation of silenes from 1,2,2,2 -tetramethyl-, 1,1,2,2-tetramethyl-, and 2-ethyl-1,2,2-trimethylphenyl - vinyldisilane" ORGANOMETALLICS, Bd. 10, Nr. 8, 1991, Seiten 2701-2706, XP000952617**
- **PATENT ABSTRACTS OF JAPAN vol. 1995, no. 10, 30. November 1995 (1995-11-30) & JP 07 173178 A (SHIN ETSU CHEM CO LTD), 11. Juli 1995 (1995-07-11)**
- **PATENT ABSTRACTS OF JAPAN vol. 1999, no. 04, 30. April 1999 (1999-04-30) & JP 11 001530 A (TORAY DOW CORNING SILICONE CO LTD), 6. Januar 1999 (1999-01-06)**
- **DATABASE CHEMABS [Online] CHEMICAL ABSTRACTS SERVICE, COLUMBUS, OHIO, US; GOEDEL, WERNER A. ET AL: "Introducing rubber into the Langmuir-Blodgett technique" retrieved from STN Database accession no. 126:226116 XP002150493 & POLYM. PREPR. (AM. CHEM. SOC., DIV. POLYM. CHEM.) (1997), 38(1), 960 ,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 202 997 B1

## Beschreibung

Die Erfindung betrifft hydrolysierbare und polymerisierbare Silane mit niedriger Viskosität, Verfahren zu deren Herstellung und deren Verwendung, insbesondere in dentalen Massen.

Aus der EP-A-0 682 033 und EP-A-0 450 624 sind hydrolysierbare und polymerisierbare Silane bekannt, die Hydroxyl- oder Carbonylgruppen bzw. Urethangruppen aufweisen. Die dort beschriebenen Moleküle sind relativ unflexibel und weisen eine hohe Kondensat-Viskosität auf, die es beispielsweise bei der Verwendung in Kompositmaterialien notwendig macht, Verdünnermonomere einzusetzen. Der Zusatz von Verdünnermonomeren hat jedoch den gravierenden Nachteil, daß die Gefahr einer erhöhten Restmonomerauslösbarkeit und damit eine erhöhte toxikologische Bedenklichkeit bestehen.

Besonders für den Dentalbereich ist es erforderlich, niedrig-viskose und flexible Moleküle einzusetzen, die dennoch nicht die Neigung besitzen, aus den formulierten Massen auszudampfen, da dadurch eine leichte Handhabbarkeit im Sinne einer leichten Ausbringbarkeit aus den Aufbewahrungsbehältnissen sowie eine gesundheitliche Unbedenklichkeit besteht. Niedrig-viskose Mischungen lassen sich ferner besser mischen und führen dadurch zu besseren, homogeneren Endprodukten.

Aufgabe der vorliegenden Erfindung ist es, organisch-modifizierte Silane bereit zu stellen, die hydrolysier- und merisierbar sind und die niedrigviskos und flexibel sind, ohne dabei aus den formulierten Massen auszudampfen, und die somit alleine zu beispielsweise Dentalmassen verarbeitet werden können, ohne daß es den Zusatz von Verdünnungsmonomeren bedarf.

Gelöst wird diese Aufgabe durch hydrolysierbare und polymerisierbare Silane der allgemeinen Formel I:

$$B\text{-}R'\text{-}U\text{-}D \qquad (I)$$

in der die Reste folgende Bedeutung haben:

B = Vinylcyclopropyl,

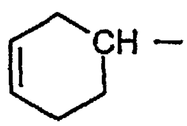 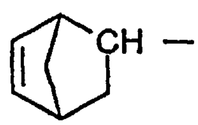

oder Methacrylatreste (einschließlich der entsprechenden Acrylatreste) aus der folgenden Gruppe:

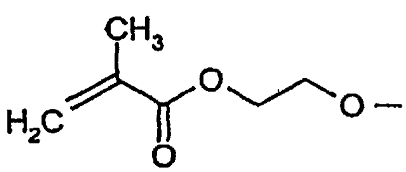

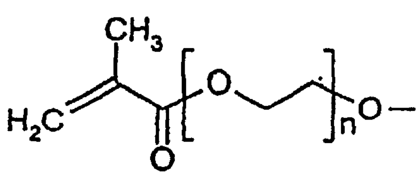

n = 2, 3, 4, 5, 6, 7, bzw. Gemische mit mittlerem n < 10

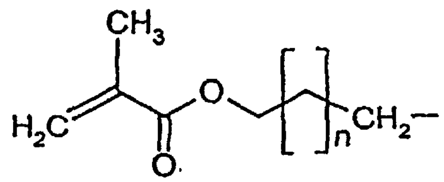

n = 0, 1, 2, 3, 4, 5, 6, 7, 8, 9, 10

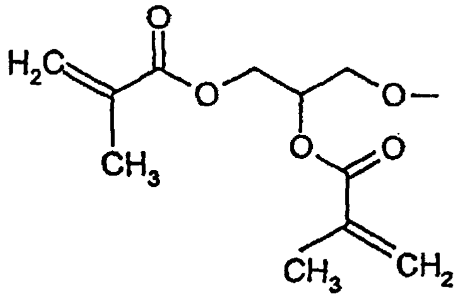

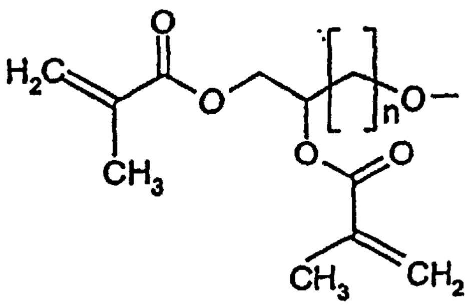

n = 1, 2, 3, 4, 5, 6, 7, 8, 9, 10

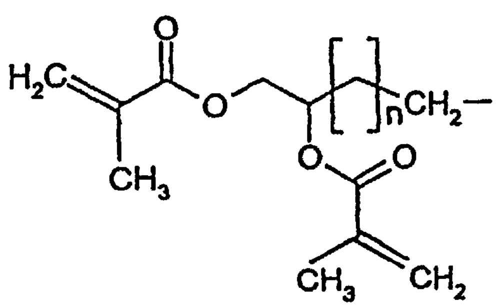

n = 0, 1, 2, 3, 4, 5, 6, 7, 8, 9, 10

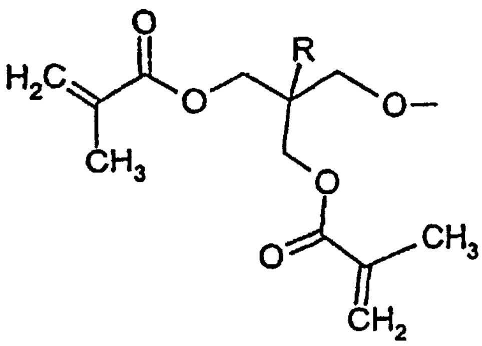

R = Alkyl, Aryl, Alkylaryl

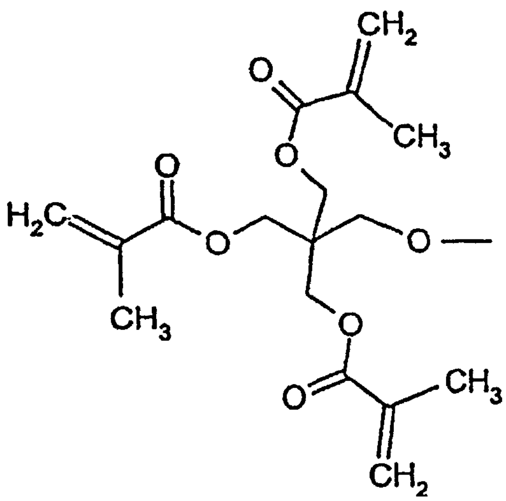

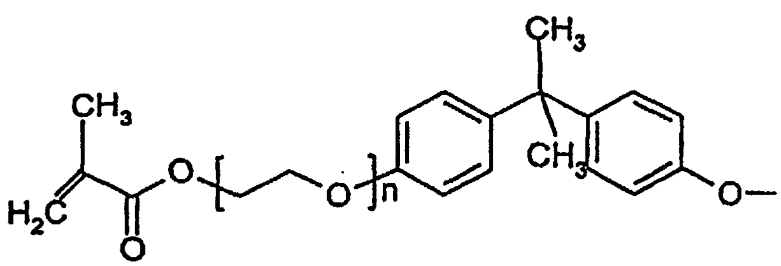

n = 1, 2, 3, 4, 5

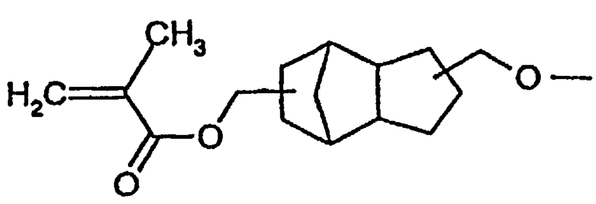 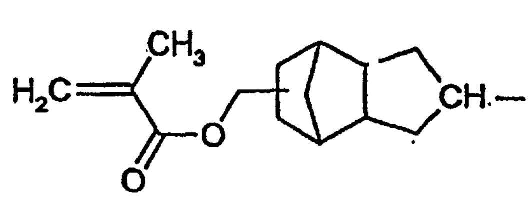 ;

R' = Alkylen, Alkenylen, Arylen, Arylenalkylen oder Alkylenarylen mit jeweils 0 bis 10 Kohlenstoffatomen, wobei diese Reste durch Sauerstoff- und Schwefelatome oder durch Aminogruppen unterbrochen sein können;

U =

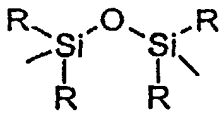

R = Alkyl, Aryl, Cycloyalkyl

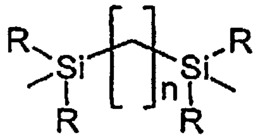

R = Alkyl, Aryl, Cycloyalkyl
n = 1, 2, 3, 4, 5, 6, 7, 8, 9, 10

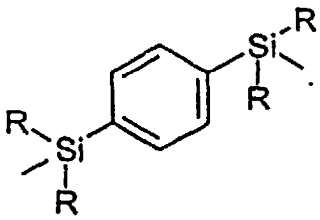

R = Alkyl, Aryl, Cycloyalkyl

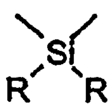

R = Alkyl, Aryl, Cycloalkyl

D = $-(CH_2)_n-Si(CH_3)_2(OC_2H_5)$, mit n = 0 bis 10
$-(CH_2)_n-Si(CH_3)(OC_2H_5)_2$, mit n = 0 bis 10
$-(CH_2)_n-Si(OCH_3)_3$, mit n = 0 bis 10
$-(CH_2)_n-Si(C_2H_5)_2(OCH_3)$, mit n = 0 bis 10
$-(CH_2)_n-Si(C_2H_5)(OCH_3)_2$, mit n = 0 bis 10
$-(CH_2)_n-Si(OC_2H_5)_3$, mit n = 0 bis 10
$-(CH_2)_n-Si(CH_3)_2(OCH_3)$, mit n = 0 bis 10
$-(CH_2)_n-Si(CH_3)(OCH_3)_2$, mit n = 0 bis 10
$-(CH_2)_n-Si(C_2H_5)_2(OC_2H_5)$, mit n = 0 bis 10
$-(CH_2)_n-Si(C_2H_5)(OC_2H_5)_2$, mit n = 0 bis 10
$-(CH_2)_n-Si(CH_3)(C_2H_5)(OCH_3)$, mit n = 0 bis 10
$-(CH_2)_n-Si(CH_3)(C_2H_5)(OC_2H_5)$, mit n = 0 bis 10
$-(CH_2)_n-Si(CH_3)(OC_2H_5)(OCH_3)$, mit n = 0 bis 10
$-(CH_2)_n-Si(C_2H_5)(OC_2H_5)(OCH_3)$, mit n = 0 bis 10
$-(CH_2)_n-Si(OC_2H_5)_2(OCH_3)$, mit n = 0 bis 10
$-(CH_2)_n-Si(OC_2H_5)(OCH_3)_2$, mit n = 0 bis 10
$-(CH_2)_n-Si(CH_3)(OC(CH_3)=CH_2)_2$, mit n = 0 bis 10
$-(CH_2)_n-Si(C_2H_5)(OC(CH_3)=CH_2)_2$, mit n = 0 bis 10
$-(CH_2)_n-Si(CH_3)_2(OC(CH_3)=CH_2)$, mit n = 0 bis 10

$-(CH_2)_n-Si(C_2H_5)_2(OC(CH_3)=CH_2)$, mit n = 0 bis 10
$-(CH_2)_n-Si(OCH_3)(OC(CH_3)=CH_2)_2$, mit n = 0 bis 10
$-(CH_2)_n-Si(OC_2H_5)(OC(CH_3)=CH_2)_2$, mit n = 0 bis 10
$-(CH_2)_n-Si(OCH_3)_2(OC(CH_3)=CH_2)$, mit n = 0 bis 10
$-(CH_2)_n-Si(OC_2H_5)_2(OC(CH_3)=CH_2)$, mit n = 0 bis 10
$-(CH_2)_n-Si(C_6H_5)(OC_2H_5)_2$, mit n = 0 bis 10
$-(CH_2)_n-Si(C_6H_5)(OCH_3)_2$, mit n = 0 bis 10
$-(CH_2)_n-Si(C_6H_5)(OCH_3)(OC_2H_5)$, mit n = 0 bis 10
$-(CH_2)_n-Si(C_6H_5)(OC(CH_3)=CH_2)_2$, mit n = 0 bis 10.

R' hat vorzugsweise die folgende Bedeutung:
Alkylen, Alkenylen, Arylen, Arylenalkylen oder Alkylenarylen mit jeweils 0 bis 5 Kohlenstoffatomen, wobei diese Reste durch Sauerstoff- und Schwefelatome oder durch Aminogruppen unterbrochen sein können.

Die erfindungsgemäßen Silane sind niedrigviskos und flexibel und dampfen aus den mit ihnen formulierten Massen nicht aus Sie können alleine, in Mischungen oder zusammen mit anderen hydrolysierbaren, kondensierbaren oder polymerisierbaren Komponenten zu kratzfesten Beschichtungen, zu , Füll-, Klebe- oder Dichtungsmassen, zu Formkörpern oder zu Einbettmaterialien verarbeitet werden.

Die erfindungsgemäßen Silane sind universell einsetzbar und können in ein anorganisch-organisches Verbundsystem, d.h. in ein anorganisch-organisches Netzwerk eingebaut werden.

Der Abstand zwischen Silicium und reaktiver Doppelbindung ist beliebig einstellbar und das Silan kann außerdem über mehrere C=C-Doppelbindungen verfügen. Ferner weist die Kette zwischen Silicium und reaktiver Doppelbindung keine zur Ausbildung von Wasserstoff-Brücken befähigte Gruppen auf.

Die Silane der Formel I sind über die Reste B polymerisierbar und über die Reste D hydrolysierbar. Über die hydrolysierbaren Gruppen kann ein anorganisches Netzwerk mit Si-O-Si-Einheiten aufgebaut werden, während die im Rest B enthaltenen Doppelbindungen unter Aufbau eines organischen Netzwerkes polymerisieren.

Hinsichtlich der Formel I und aller nachfolgenden Formeln gelten ganz allgemein die nachstehenden Restedefinitionen.

Die Alkylreste sind beispielsweise geradkettige, verzweigte oder cyclische Reste mit 1 bis 20, insbesondere mit 1 bis 10 Kohlenstoffatomen und vorzugsweise niedere Alkylreste mit 1 bis 6, besonders bevorzugt mit 1 bis 4 Kohlenstoffatomen. Spezielle Beispiele sind Methyl, Ethyl, n-Propyl, i-Propyl, n-Butyl, s-Butyl, i-Butyl, t-Butyl, n-Pentyl, n-Hexyl, Cyclohexyl, 2-Ethylhexyl, Dodecyl und Octadecyl.

Die Alkenylreste sind beispielsweise geradkettige, verzweigte oder cyclische Reste mit 2 bis 20, bevorzugt mit 2 bis 10 Kohlenstoffatomen und vorzugsweise niedere Alkenylreste mit 2 bis 6 Kohlenstoffatomen, wie Vinyl, Allyl und 2-Butenyl.

Bevorzugte Arylreste haben 6 - 20, besonders bevorzugt 6 - 15 Kohlenstoffatome. Bevorzugte Acylreste haben 1 - 15 Köhlenstoffatome, bevorzugt 1 - 10 Kohlenstoffatome. Bevorzugte Arylalkyl- und Alkylarylreste weisen 7 - 25, bevorzugt 7 - 15 Kohlenstoffatome auf. Bevorzugte Alkylenreste haben 2 - 15, besonders bevorzugt 2 - 10 C-Atome. Bevorzugte Arylenreste haben 6 - 25, besonders bevorzugt 6 - 15 C-Atome. Bevorzugte Alkylenarylenreste haben 7 - 25, besonders bevorzugt 7 - 15 C-Atome. Bevorzugte Alkoxy, Acyloxy-, Alkylcarbonyl oder Alkoxycarbonylreste haben 1 - 15, besonders bevorzugt 1 - 10 C-Atome. Bevorzugte Alkylamino und Dialkylaminoreste haben 1 - 15, besonders bevorzugt 1- 10 C-Atome.

Bevorzugte Arylreste sind Phenyl, Biphenyl und Naphthyl. Die Alkoxy-, Acyloxy-, Alkylamino-, Dialkylamino-, Alkylcarbonyl-, Alkoxycarbonyl-, Arylalkyl-, Alkylaryl-, Alkylen- und Alkylen- und Alkylenarylenreste leiten sich vorzugsweise von den oben genannten Alkyl- und Arylresten ab. Spezielle Beispiele sind Methoxy, Ethoxy, n-und i-Propoxy, n-, i-, s- und t-Butoxy, Monomethylamino, Monoethylamino, Dimethylamino, Diethylamino, N-Ethylanilino, Acetyloxy, Propionyloxy, Methylcarbonyl, Ethylcarbonyl, Methoxycarbonyl, Ethoxycarbonyl, Benzyl, 2-Phenylethyl und Tolyl.

Die genannten Reste können gegebenenfalls einen oder mehrere Substituenten tragen, beispielsweise Halogen, Alkyl, Hydroxyalkyl, Alkoxy, Aryloxy, Alkylcarbonyl, Alkoxycarbonyl, Furfuryl, Tetrahydrofurfuryl, Amino, Monoalkylamino, Dialkylamino, Trialkylammonium, Amido, Hydroxy, Formyl, Carboxy, Mercapto, Cyano, Isocyanato, Nitro, Epoxy, $SO_3H$ oder $PO_4H_2$.

Unter den Halogenen sind Fluor, Chlor und Brom und insbesondere Chlor bevorzugt.

Der Rest hat die folgende Bedeutung:
Vinylcyclopropyl,

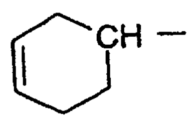 , 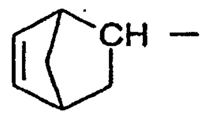

oder Methacrylatreste (einschließlich der entsprechenden Acrylatreste aus der folgenden Gruppe:

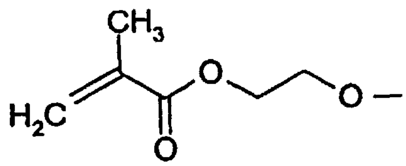

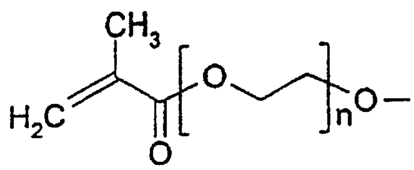

n = 2, 3, 4, 5, 6, 7, bzw. Gemische mit mittlerem n < 10

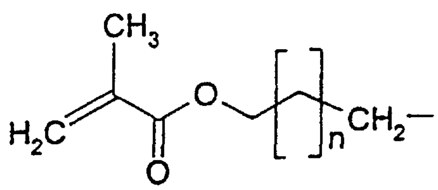

n = 0, 1, 2, 3, 4, 5, 6, 7, 8, 9, 10

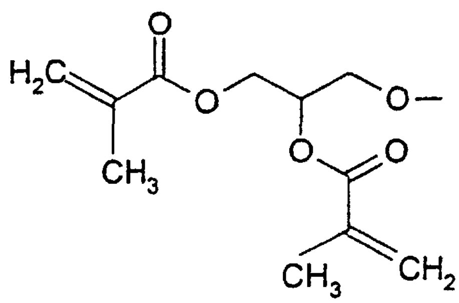

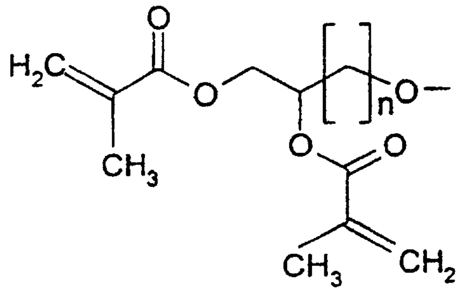

n = 1, 2, 3, 4, 5, 6, 7, 8, 9, 10

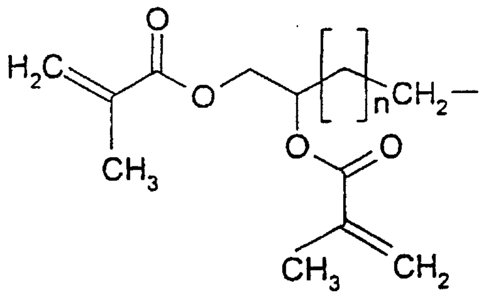

n = 0, 1, 2, 3, 4, 5, 6, 7, 8, 9, 10

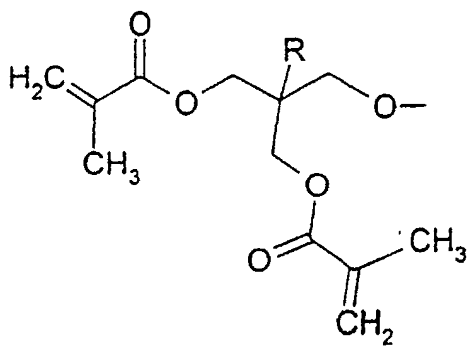

R = Alkyl, Aryl, Alkylaryl

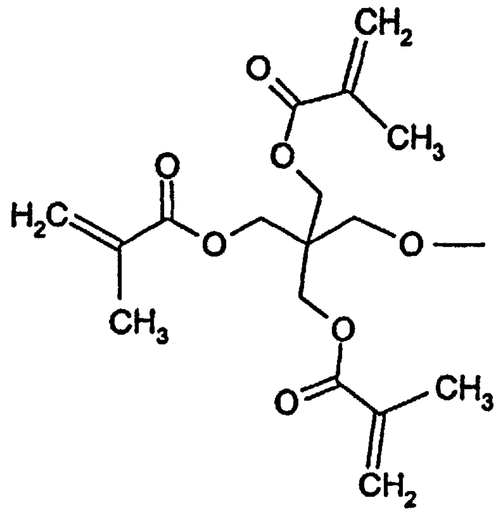

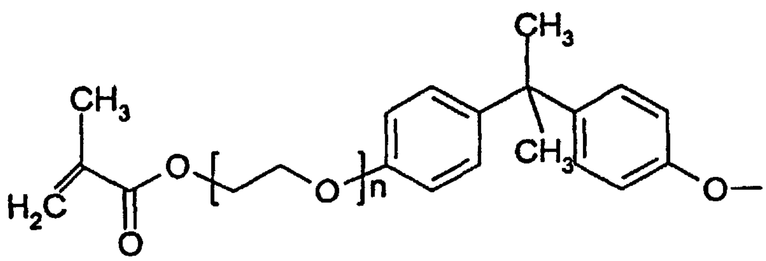

n = 1, 2, 3, 4, 5

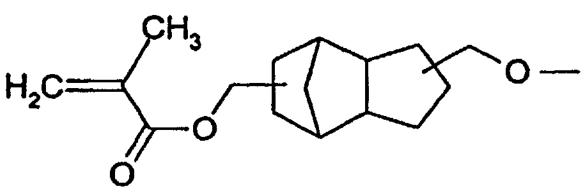 , 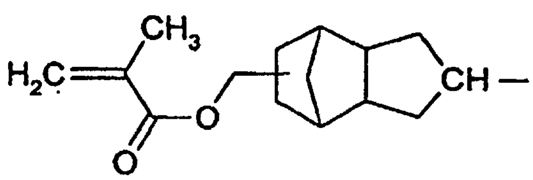

Über seine freien Valenzen ist der Rest B jeweils mit einer Gruppe -R'- verknüpft.

Der Rest U kann die folgenden Bedeutungen haben.

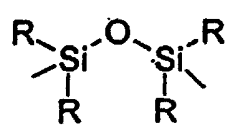

R = Alkyl, Aryl, Cycloyalkyl

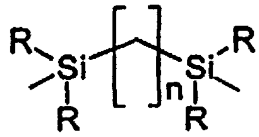

R = Alkyl, Aryl, Cycloyalkyl
n = 1, 2, 3, 4, 5, 6, 7, 8, 9, 10

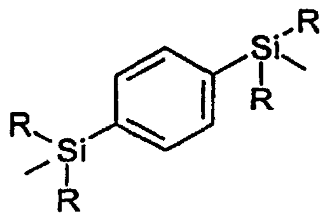

R = Alkyl, Aryl, Cycloyalkyl

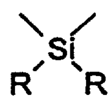

R = Alkyl, Aryl, Cycloalkyl

Die erfindungsgemäßen Silane können beispielsweise dadurch hergestellt werden, daß man in an sich bekannter Weise ein C=C-ungesättigtes Silan einer äquimolaren Additionsreaktion mit einer Verbindung der allgemeinen Formel H-U-H unterwirft, wobei das äquimolare Additionsprodukt nötigenfalls physikalisch separiert wird. U hat dabei die obige Bedeutung. Das äquimolare Additionsprodukt
wird in ein erfindungsgemäßes Derivat überführt, indem man es einer weiteren Additionsreaktion unterwirft.

Im Folgenden werden anhand von konkreten Reaktionsgleichungen die Syntheseprinzipien näher erläutert:

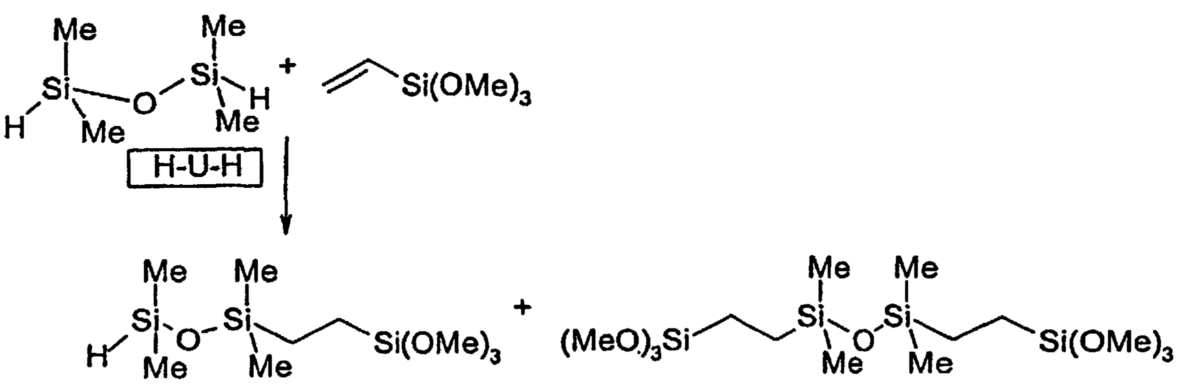


destillative Abtrennung

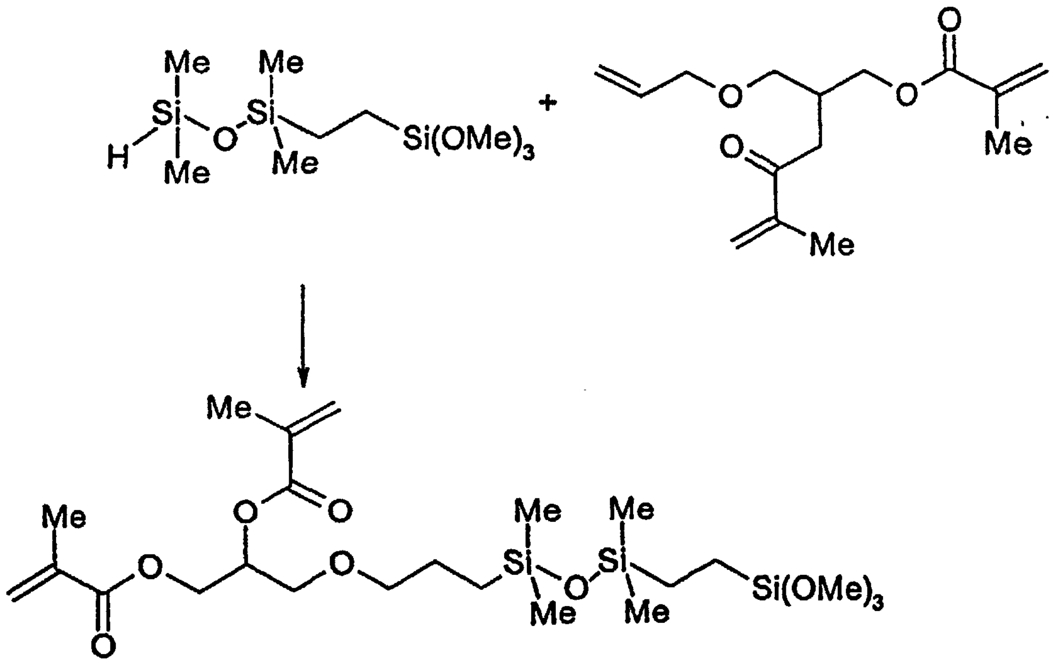

oder

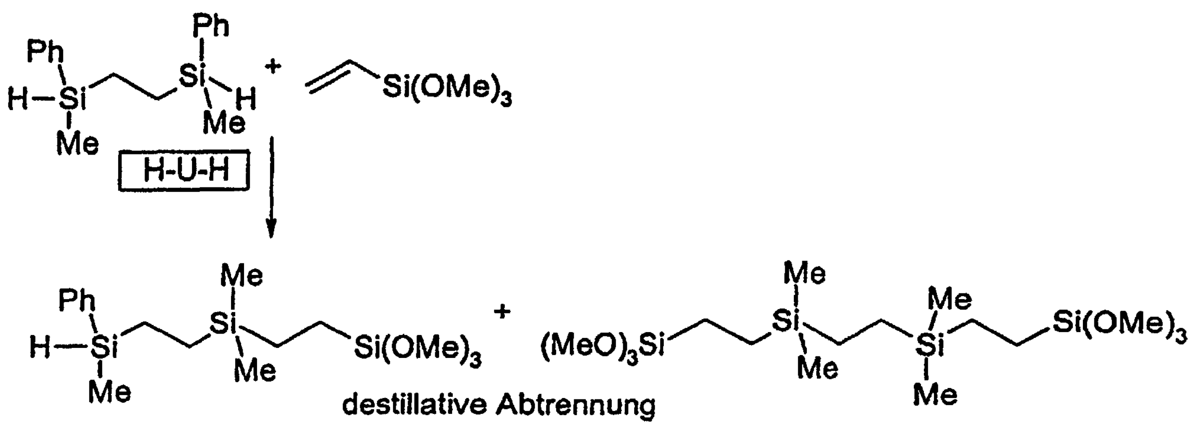

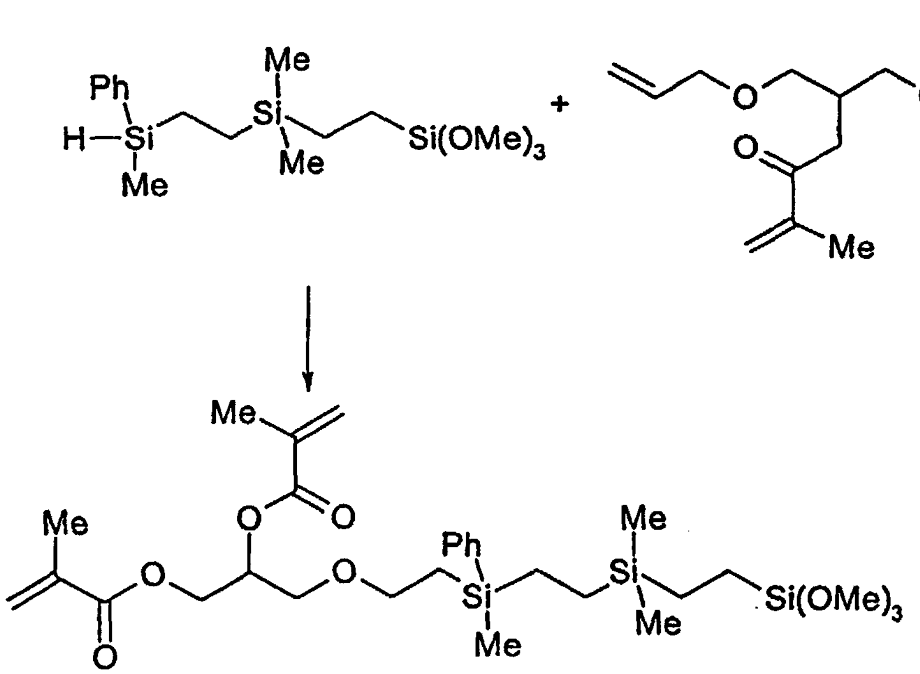

Bei allen diesen Reaktionstypen ist eine mehrfache, bis zu vierfache Addition der entsprechenden Silane möglich.

Der Rest kann die folgende Bedeutung haben.

- $(CH_2)_n$-$Si(CH_3)_2(OC_2H_5)$, mit n = 0 bis 10
- $(CH_2)_n$-$Si(CH_3)(OC_2H_5)_2$, mit n = 0 bis 10
- $(CH_2)_n$-$Si(OCH_3)_3$, mit n = 0 bis 10
- $(CH_2)_n$-$Si(C_2H_5)_2(OCH_3)$, mit n = 0 bis 10
- $(CH_2)_n$-$Si(C_2H_5)(OCH_3)_2$, mit n = 0 bis 10
- $(CH_2)_n$-$Si(OC_2H_5)_3$, mit n = 0 bis 10
- $(CH_2)_n$-$Si(CH_3)_2(OCH_3)$, mit n = 0 bis 10
- $(CH_2)_n$-$Si(CH_3)(OCH_3)_2$, mit n = 0 bis 10
- $(CH_2)_n$-$Si(C_2H_5)_2(OC_2H_5)$, mit n = 0 bis 10
- $(CH_2)_n$-$Si(C_2H_5)(OC_2H_5)_2$, mit n = 0 bis 10
- $(CH_2)_n$-$Si(CH_3)(C_2H_5)(OCH_3)$, mit n 0 bis 10
- $(CH_2)_n$-$Si(CH_3)(C_2H_5)(OC_2H_5)$, mit n 0 bis 10
- $(CH_2)_n$-$Si(CH_3)(OC_2H_5)(OCH_3)$, mit n = 0 bis 10
- $(CH_2)_n$-$Si(C_2H_5)(OC_2H_5)(OCH_3)$, mit n = 0 bis 10
- $(CH_2)_n$-$Si(OC_2H_5)_2(OCH_3)$, mit n = 0 bis 10
- $(CH_2)_n$-$Si(OC_2H_5)(OCH_3)_2$, mit n = 0 bis 10
- $(CH_2)_n$-$Si(CH_3)(OC(CH_3)=CH_2)_2$, mit n = 0 bis 10
- $(CH_2)_n$-$Si(C_2H_5)(OC(CH_3)=CH_2)_2$, mit n = 0 bis 10
- $(CH_2)_n$-$Si(CH_3)_2(OC(CH_3)=CH_2)$, mit n = 0 bis 10
- $(CH_2)_n$-$Si(C_2H_5)_2(OC(CH_3)=CH_2)$, mit n = 0 bis 10
- $(CH_2)_n$-$Si(OC_2H_5)(OC(CH_3)=CH_2)_2$, mit n = 0 bis 10
- $(CH_2)_n$-$Si(OCH_3)(OC(CH_3)=CH_2)_2$, mit n = 0 bis 10
- $(CH_2)_n$-$Si(OC_2H)_3(OC(CH_3)=CH_2)$, mit n = 0 bis 10
- $(CH_2)_n$-$Si(OC_2H_5)_2(OC(CH_3)=CH_2)$, mit n = 0 bis 10
- $(CH_2)_n$-$Si(C_6H_5)(OC_2H_5)_2$, mit n = 0 bis 10
- $(CH_2)_n$-$Si(C_6H_5)(OCH_3)_2$, mit n = 0 bis 10
- $(CH_2)_n$-$Si(C_6H_5)(OCH_3)(OC_2H_5)$, mit n = 0 bis 10
- $(CH_2)_n$-$Si(C_6H_5)(OC(CH_3)=CH_2)_2$, mit n = 0 bis 10.

Die erfindungsgemäßen Silane sind stabile Verbindungen und können entweder alleine oder zusammen mit

anderen hydrolysierbaren, kondensierbaren und/oder polymerisierbaren Komponenten zu Kieselsäurepolykondensaten oder zu Kieselsäureheteropolykondensaten verarbeitet werden, deren endgültige Härtung dann durch Polymerisation der C=C-Doppelbindungen erfolgt.

Die erfindungsgemäßen Silane können aber auch alleine oder zusammen mit anderen hydrolysierbaren, kondensierbaren und/oder polymerisierbaren Komponenten zu Polymerisaten verarbeitet werden, die durch anschließende hydrolytische Kondensation verfestigt werden können.

Kieselsäure(hetero)polykondensate, die mit organischen Gruppen modifiziert sind, sowie Verfahren zu deren Herstellung (beispielsweise ausgehend von hydrolytisch kondensierbaren Organosilanen nach dem Sol-Gel-Prozeß) sind in großer Zahl bekannt. Derartige Kondensate finden, wie eingangs schon erwähnt, für die verschiedensten Zwekke Verwendung, beispielsweise als Formmassen oder als Lacke für Überzüge. Aufgrund der vielfältigen Anwendungsmöglichkeiten dieser Substanzklasse besteht aber auch ein ständiges Bedürfnis nach Modifizierung der bereits bekannten Kondensate, zum einen, um dadurch neue Anwendungsgebiete zu erschließen und zum anderen, um deren Eigenschaften für bestimmte Verwendungszwecke noch weiter zu optimieren.

Die erfindungsgemäßen Silane sind im basischen oder sauren Milieu hydrolysierund kondensierbar, ohne daß dadurch eine Verknüpfung der C=C-Doppelbindungen erfolgt. Dadurch ist es möglich, die erfindungsgemäßen Silane durch hydrolytische Kondensation in ein anorganisch-organisches Netzwerk einzubauen. Die erfindungsgemäßen Silane enthalten hydrolysierbare Gruppen, beispielsweise Alkoxygruppen, so daß damit ein anorganisches Netzwerk (Si-O-Si-Einheiten) aufgebaut werden kann, während die im Rest B enthaltenen C=C-Doppelbindungen unter Aufbau eines organischen Netzwerkes polymerisiert werden können. Dadurch ist es möglich, organisch modifizierte, hydrolysier- und kondensierbare Silane in Beschichtungs-, Füll-, Klebe- und Dichtungsmassen, in Formkörpern und Einbettmassen nach dem Stand der Technik durch die erfindungsgemäßen Silane zu ersetzen.

Zum Aufbau des anorganischen Netzwerkes werden die erfindungsgemäßen Silane, gegebenenfalls unter Zusatz anderer cokondensierbarer Komponenten hydrolysiert und polykondensiert. Die Polykondensation erfolgt vorzugsweise nach dem Sol-Gel-Verfahren, wie es beispielsweise in den DE-A-2 758 414, DE-A-2 758 415, DE-A-3 011 761, DE-A-3 826 715 und DE-A-3 835 968 beschrieben ist.

Zum Aufbau des organischen Netzwerkes werden die erfindungsgemäßen Silane gegebenenfalls unter Zusatz anderer copolymerisierbarer Komponenten polymerisiert. Die Polymerisation kann beispielsweise thermisch, redoxinduziert, kovalent-nucleophil und/oder photochemisch unter Einsatz von Methoden erfolgen, wie sie beispielsweise in den DE-A-3 143 820, DE-A-3 826 715 und DE-A-3 835 968 beschrieben sind.

Als weitere polymerisierbare Komponenten können Verbindungen zugesetzt werden, die radikalisch und/oder ionisch polymerisierbar sind. Radikalisch polymerisierbare Verbindungen, die zugesetzt werden können, sind beispielsweise solche mit C=C-Doppelbindungen, wie. Acrylate, Vinylcyclopropane oder Methacrylate, wobei die Polymerisation über die C=C-Doppelbindungen, gegebenenfalls unter Einbezug des Ringes erfolgt. Ionisch polymerisierbare Verbindungen, die zugesetzt werden können, enthalten beispielsweise Ringsysteme, die kationisch ringöffnend polymerisierbar sind, wie Spiroorthoester, Spiroorthocarbonate, bicyclische Spiroorthoester, Mono- oder Oligoepoxide oder -oxetane oder Spiro-Silane, wie sie aus der DE-C-4 125 201 bekannt sind.

Es können aber auch Verbindungen zugesetzt werden, die sowohl ionisch als auch radikalisch polymerisierbar sind, wie Methacryloyl-Spiroorthoester. Diese sind radikalisch über die C=C-Doppelbindung und kationisch unter Ringöffnung polymerisierbar. Die Herstellung dieser Systeme ist beispielsweise im Journal f. prakt. Chemie, Band 330, Heft 2, 1988, S. 316-318 beschrieben. Desweiteren können die erfindungsgemäßen Silane in Systemen eingesetzt werden, wie sie beispielsweise in der DE-A-4 405 261 beschrieben sind.

Ferner ist es möglich, andere bekannte, silan-gebundene cyclische Systeme zuzusetzen, die mit einpolymerisiert werden können. Solche Systeme sind beispielsweise solche, die Epoxide enthalten. Deartige Systeme sind bei der Herstellung der Spiro-Silane der DE-C-4 125 201 beschrieben.

Die erfindungsgemäßen Silane stellen hoch reaktive Systeme dar, die zu Poly(hetero)kondensaten führen, die beispielsweise bei UV-Bestrahlung innerhalb kürzester Zeit zu mechanisch stabilen Überzügen oder Form- bzw. Füllkörpem führen. Die erfindungsgemäßen Silane sind über einfache Additionsreaktionen herstellbar und können durch geeignete Auswahl der Ausgangsverbindungen eine variierbare Anzahl reaktiver Gruppen unterschiedlicher Funktionalität aufweisen.

Bei Anwesenheit von zwei oder mehr C=C-Doppelbindungen im Rest B ist die Ausbildung eines dreidimensionalen, organischen Netzwerkes möglich. Über den Abstand zwischen dem Si-Atom und dem Rest B, d.h. über die Kettenlänge, und über die Anwesenheit weiterer funktioneller Gruppen in dieser Kette, können die mechanischen Eigenschaften. (z.B. Flexibilität) und die physikalisch-chemischen Eigenschaften (z.B. Adsorption, Brechzahl, Haftung) der Poly(hetero)kondensate beeinflußt werden. Durch die Ausbildung eines anorganischen Netzwerkes können je nach Art und Anzahl der hydrolysierbaren Gruppen (z.B. Alkoxygruppen) siliconoder glasartige Eigenschaften der Poly (hetero)kondensate eingestellt werden.

Die erfindungsgemäßen Silane besitzen relativ hohe Molekulargewichte und dementsprechend eine verminderte Flüchtigkeit gegenüber reinen (Meth)Acrylatmonomeren, so daß die toxische Gefährdung während der Verarbeitung und Applikation geringer ist. Bei der anorganischen und/oder organischen Vernetzung bilden sich Polysiloxane mit nochmals erniedrigter Flüchtigkeit, die damit das Toxizitätsproblem der Acrylatkomponenten völlig beheben.

Berücksichtigt man dann noch die Variationsmöglichkeiten der cokondensierbaren und copolymerisierbaren Komponenten, so wird offenbar, daß über die erfindungsgemäßen Silane Kieselsäure(hetero)polykondensate zur Verfügung gestellt werden, die in vielfältiger Weise an vorgegebene Einsatzgebiete angepaßt werden können und die deshalb auf allen Gebieten, auf denen bereits bisher Kieselsäure(hetero)polykondensate eingesetzt wurden, Verwendung finden können, aber auch neue Verwendungsmöglichkeiten eröffnen, beispielsweise auf dem Gebiet der Optik, der Elektronik, der Medizin, insbesondere der Dentalmedizin, der Optoelektronik und der Verpackungsmittel für Lebensmittel.

Die erfindungsgemäßen Silane können entweder als solche verwendet werden oder in Zusammensetzungen, die zusätzlich an den Verwendungszweck angepaßte Additive enthalten, beispielsweise übliche Lackadditive, Lösungsmittel, Füllstoffe, Photoinitiatoren, thermische Initiatoren, Verlaufsmittel und Pigmente.

Die erfindungsgemäßen Silane oder die silanhaltigen Zusammensetzungen eignen sich beispielsweise zur Herstellung von Beschichtungs-, Füllstoff-, oder Bulkmaterialien, von Klebstoffen und Spritzgußmassen, von Fasern, Partikeln, Folien, Haftvermittlern, von Abformmassen und von Einbettmaterialien.

Beschichtungen und Formkörper aus den erfindungsgemäßen Silanen haben den Vorteil, daß sie photochemisch strukturierbar sind. Spezielle Anwendungsgebiete sind beispielsweise die Beschichtung von Substraten aus Metall, Kunststoff, Papier, Keramik (durch Tauchen, Gießen, Streichen, Spritzen, elektrostatisches Spritzen, Elektrotauchlackierung), der Einsatz für optische, optoelektrische oder elektronische Komponenten, die Herstellung von Füllstoffen, die Herstellung von kratzfesten, abriebfesten Korrosionsschutzbeschichtungen von Formkörpern, beispielsweise durch Spritzguß, Formgießen, Pressen, Rapid-Prototyping oder Extrusion, und die Herstellung von Composites, beispielsweise mit Fasern, Füllstoffen oder Geweben.

Neben den erfindungsgemäßen Silanen der Formel I können noch weitere hydrolytisch kondensierbare Verbindungen des Siliciums, des Bors, des Aluminiums, des Phosphors, des Zinns, des Bleis, der Übergangsmetalle, der Lanthaniden oder Actiniden eingesetzt werden. Diese Verbindungen können entweder als solche oder bereits in vorkondensierter Form zur Herstellung der Polykondensate herangezogen werden. Bevorzugt ist es, wenn mindestens 10 Mol-%, insbesondere mindestens 80 Mol-% und speziell mindestens 90 Mol-%, auf der Basis monomerer Verbindungen, der zur Herstellung der Kieselsäure(hetero)polykondensate herangezogenen Ausgangsmaterialien Siliciumverbindungen sind.

Ebenso ist es bevorzugt, wenn den Kieselsäure(hetero)polykondensaten mindestens 5 Mol-%, beispielsweise 25 bis 100 Mol-%, insbesondere 50 bis 100 Mol-%, und speziell 75 bis 100 Mol-%, jeweils auf der Basis monomerer Verbindungen, an einem oder mehreren der erfindungsgemäßen Silane zugrunde liegen.

Unter den von Silanen der allgemeinen Formel I verschiedenen, hydrolytisch kondensierbaren Siliciumverbindungen, die gegebenenfalls eingesetzt werden können, sind solche der allgemeinen Formel VI besonders bevorzugt:

$$R_e(R^{10}Z')_fSiX_{4-(e+f)} \qquad \text{(VI)}$$

in der die Reste und Indices die folgende Bedeutung haben:

X = Wasserstoff, Halogen, Hydroxy, Alkoxy, Acyloxy, Alkylcarbonyl, Alkoxycarbonyl oder $NR''_2$;
R = Alkyl, Alkenyl, Aryl, Alkylaryl oder Arylalkyl;
R"= Wasserstoff, Alkyl oder Aryl;
$R^{10}$= Alkylen oder Alkenylen, wobei diese Reste durch Sauerstoff- oder Schwefelatome oder -NH-Gruppen unterbrochen sein können;
Z' = Halogen oder eine gegebenenfalls substituierte Amino-, Amid-, Aldehyd-, Alkylcarbonyl-, Carboxy-, Mercapto-, Cyano-, Alkoxy-, Alkoxycarbonyl-, Sulfonsäure-, Phosphorsäure-, Acryloxy-, Methacryloxy-, Epoxy- oder Vinylgruppe;
e = 0, 1, 2 oder 3;
f= 0, 1, 2 oder 3, mit e + f = 1, 2 oder 3

Solche Silane sind beispielsweise in der DE-C-34 07 087 beschrieben.

Spezielle Beispiele für hydrolytisch kondensierbare Silane der allgemeinen Formel VI sind: $CH_3$-Si-$Cl_3$, $CH_3$-Si-$(OC_2H_5)_3$, $C_2H_5$-Si-$Cl_3$, $C_2H_5$-Si-$(OC_2H_5)_3$, $CH_2$=CH-Si-$(OC_2H_5)_3$, $CH_2$=CH-Si$(OC_2H_4OCH_3)_3$, $(CH_3)_2$-Si-$Cl_2$, $CH_2$=CH-Si-$(OOCCH_3)_3$, $(CH_3)_2$-Si-$(OC_2H_5)_2$, $(C_2H_5)_3$-Si-Cl, $(C_2H_5)_2$-Si-$(OC_2H_5)_2$, $(CH_3)_2(CH_2$=CH) -Si-$Cl_2$, $(CH_3)_3$-Si-Cl, (t-$C_4H_9$)$(CH_3)_2$-Si-Cl, $(CH_3O)_3$-Si-$C_3H_5$-NH-$C_2H_4$-NH-$C_2H_4$-$NH_2$, $(CH_3O)_3$-Si-$C_3H_6$-SH,

$(CH_3O)_3$-Si-$C_3H_6$-NH-$C_2H_4$-$NH_2$, $(CH_3O)_3$-Si-$C_3H_6$-Cl, $(CH_3O)_3$-Si-$C_3H_5$-O-C(O)-C$(CH_3)$=$CH_2$, $(CH_3)_2$ $(CH_2$=CH-$CH_2)$-Si-Cl, $(C_2H_5O)_3$-Si-$C_3H_6$-$NH_2$, $(C_2H_5O)_3$-Si-$C_3H_6$CN, $(CH_3O)_3$-Si-$(CH_2)_2$

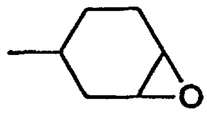
,

$(CH_3O)_3$-Si-$C_3H_6$-O-$CH_2$

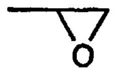
.

Die erfindungsgemäßen Silane müssen für die Weiterverarbeitung zu den Poly(hetero)kondensaten nicht unbedingt extra isoliert werden. Es ist auch möglich, in einem Eintopf-Verfahren, diese Silane zunächst herzustellen und dann - gegebenenfalls nach Zusatz weiterer hydrolysierbarer Verbindungen - hydrolytisch zu kondensieren.

Unter den von Silanen der allgemeinen Formel I verschiedenen, hydrolytisch kondensierbaren Siliciumverbindungen, die gegebenenfalls, noch eingesetzt werden können, sind solche der allgemeinen Formel VIII ebenfalls besonders bevorzugt:

$$Y_n\ Si\ X_m\ R_{4-(n+m)} \qquad (VIII)$$

in der und R die obige und Y, n und m die folgende Bedeutung haben:

- Y= ein Substituent, der einen substituierten oder unsubstituierten 1,4,6-Trioxaspiro-[4,4]-nonanrest enthält;
- n= 1, 2 oder 3;
- m= 1, 2 oder 3, mit $n + m \leqq 4$.

Diese Spiro-Silane sind über die Reste X hydrolysierbar und über die Reste Y polymerisierbar und sie sind in der DE-C-4 125 201 ausführlichst beschrieben.

Unter den von Silanen der allgemeinen Formel I verschiedenen, hydrolytisch kondensierbaren Siliciumverbindungen, die gegebenenfalls eingesetzt werden können, sind solche der allgemeinen Formel IX ebenfalls bevorzugt:

$$G\{A-(Z)_d-R^{20}(R^{21})-R'-SiX_aR_b\}_c \qquad (IX)$$

in der die Reste und indices die folgende Bedeutung haben:

- X = Wasserstoff, Halogen, Hydroxy, Alkoxy, Acyloxy, Alkylcarbonyl, Alkoxycarbonyl oder $NR''_2$;
- R = Alkyl, Alkenyl, Aryl, Alkylaryl oder Arylalkyl;
- R' = Alkylen, Alkenylen, Arylen, Arylenalkylen oder Alkylenarylen mit jeweils 0 bis 10 Kohlenstoffatomen, wobei diese Reste durch Sauerstoff- und Schwefelatome oder durch Aminogruppen unterbrochen sein können;
- R" = Wasserstoff, Alkyl oder Aryl;
- G = ein ein- bis vierwertiger, geradkettiger oder verzweigter organischer Rest mit mindestens einer C=C-Doppelbindung und 4 bis 50 Kohlenstoffatomen;
- A = O, S oder NH für d = 1 und
  - Z = CO und
  - $R^{20}$ = Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und
  - $R^{21}$ = COOH;

oder

A = O, S oder NH für d = 1 und

Z = CO und
$R^{20}$= Alkylen, Arylen oder Alkylenarylen mit jeweils 1 bis 10 Kohlenstoffatomen, wobei diese Rest durch Sauerstoff- und Schwefelatome oder durch Aminogruppenunterbrochen sein können, und

$R^{21}$ = H;

oder

A = O, S, NH oder COO für d = 1 und

Z = CHR, mit R gleich H, Alkyl, Aryl oder Alkylaryl, und
$R^{20}$= Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und
$R^{21}$= OH;

oder

A = O, S, NH oder COO für d = 0 und

$R^{20}$ = Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und
$R^{21}$ = OH;

oder

A = S für d = 1 und

Z = CO und
$R^{20}$= N und
$R^{21}$= H;

a= 1, 2 oder 3;
b = 0, 1 oder 2;
a + b = 3;
c = 1, 2, 3 oder 4;
d = 0 oder 1.

Die Silane der Formel IX sind über die Reste G polymerisierbar und über die Reste X hydrolysierbar. Über die hydrolysierbaren Gruppen kann ein anorganisches Netzwerk mit Si-O-Si-Einheiten aufgebaut werden, während die im Rest G enthaltenen Doppelbindungen unter Aufbau eines organischen Netzwerkes polymerisieren.

Für a = 2 bzw. b = 2 können die Reste X und R jeweils dieselbe oder eine unterschiedliche Bedeutung haben.

Unter den gegebenenfalls eingesetzten hydrolysierbaren Aluminiumverbindungen sind diejenigen besonders bevorzugt, die die allgemeine Formel $AlR^0_3$ aufweisen, in der die Reste $R^0$, die gleich oder verschieden sein können, ausgewählt sind aus Halogen, Alkoxy, Alkoxycarbonyl und Hydroxy. Hinsichtlich der näheren (bevorzugten) Definitionen dieser Reste kann auf die Ausführungen im Zusammenhang mit den geeigneten hydrolysierbaren Siliciumverbindungen verwiesen werden. Die soeben genannten Gruppen können auch ganz oder teilweise durch Chelatliganden (beispielsweise Acetylaceton oder Acetessigsäureester, Essigsäure) ersetzt sein.

Besonders bevorzugte Aluminiumverbindungen sind Aluminiumalkoxide und -halogenide. In diesem Zusammenhang können als konkrete Beispiele genannt werden:

$$Al(OCH_3)_3,\ Al(OC_2H_5)_3,\ Al(O\text{-}n\text{-}C_3H_7)_3,\ Al(O\text{-}i\text{-}C_3H_7)_3,\ Al(OC_4H_9)_3,\ Al(O\text{-}i\text{-}C_4H_9)_3,$$

$$Al(O\text{-}s\text{-}C_4H_9)_3,\ AlCl_3,\ AlCl\text{-}(OH)_2.$$

Bei Raumtemperatur flüssige Verbindungen, wie beispielsweise Aluminiumsek.-butylat und Aluminium-isopropylat, werden besonders bevorzugt.

Geeignete hydrolysierbare Titan- oder Zirkoniumverbindungen, die gegebenenfalls eingesetzt werden können, sind solche der allgemeinen Formel M $X_yR_z$, in der M Ti oder Zr bedeutet, y eine ganze Zahl von 1 bis 4 ist, insbesondere 2 bis 4, z für 0, 1, 2 oder 3 steht, vorzugsweise für 0, 1 oder 2, und X und R wie im Falle der allgemeinen Formel I definiert sind. Dies gilt auch für die bevorzugten Bedeutungen. Besonders bevorzugt ist es, wenn y gleich 4 ist.

Wie im Falle der obigen Al-Verbindungen können auch komplexierte Ti- oder Zr-Verbindungen eingesetzt werden. Zusätzliche bevorzugte Komplexbildner sind hier Acrylsäure und Methacrylsäure.

Konkrete Beispiele für einsetzbare Zr- und Ti-Verbindungen sind die folgenden:

$TiCl_4$, $Ti(OC_2H_5)_4$, $Ti(OC_3H_7)_4$, $Ti(O\text{-}i\text{-}C_3H_7)_4$, $Ti(OC_4H_9)_4$, Ti(2-ethylhexoxy)$_4$, $ZrCl_4$,

$Zr(OC_2H_5)_4$, Zr-$(OC_3H_7)_4$, $Zr(O\text{-}i\text{-}C_3H_7)_4$, $Zr(OC_4H_9)_4$, Zr(2-ethylhexoxy)$_4$, $ZrOCl_2$.

Weitere hydrolysierbare Verbindungen, die zur Herstellung der Polyheterokondensate eingesetzt werden können, sind beispielsweise Bortrihalogenide und Borsäureester, wie $BCl_3$, $B(OCH_3)_3$ und $B(OC_2H_5)_3$, Zinntetrahalogenide und Zinntetraalkoxide, wie $SnCl_4$ und $Sn(OCH_3)_4$, und Vanadylverbindungen, wie $VOCl_3$ und $VO(OCH_3)_3$.

Wie bereits erwähnt, kann die Herstellung der Poly(hetero)kondensate in auf diesem Gebiet üblicher Art und Weise erfolgen. Werden praktisch ausschließlich Siliciumverbindungen eingesetzt, kann die hydrolytische Kondensation in den meisten Fällen dadurch erfolgen, daß man den zu hydrolysierenden Siliciumverbindungen, die entweder als solche oder gelöst in einem geeigneten Lösungsmittel vorliegen, das erforderliche Wasser bei Raumtemperatur oder unter leichter Kühlung direkt zugibt (vorzugsweise unter Rühren und in Anwesenheit eines Hydrolyse- und Kondensationskatalysators) und die resultierende Mischung daraufhin einige Zeit (ein bis mehrere Stunden) rührt.

Bei Anwesenheit reaktiver Verbindungen von Al, Ti oder Zr empfiehlt sich in der Regel eine stufenweise Zugabe des Wassers. Unabhängig von der Reaktivität der anwesenden Verbindungen erfolgt die Hydrolyse in der Regel bei Temperaturen zwischen -20 und 130°C, vorzugsweise zwischen 0 und 30° C bzw. dem Siedepunkt des gegebenenfalls eingesetzten Lösungsmittels. Wie bereits angedeutet, hängt die beste Art und Weise der Zugabe von Wasser vor allem von der Reaktivität der eingesetzten Ausgangsverbindungen ab. So kann man beispielsweise die gelösten Ausgangsverbindungen langsam zu einem Überschuß an Wasser tropfen, oder man gibt Wasser in einer Portion oder portionsweise den gegebenenfalls gelösten Ausgangsverbindungen zu. Es kann auch nützlich sein, das Wasser nicht als solches zuzugeben, sondern mit Hilfe von wasserhaltigen organischen oder anorganischen Systemen in das Reaktionssystem einzutragen. Als besonders geeignet hat sich in vielen Fällen die Eintragung der Wassermenge in das Reaktionsgemisch mit Hilfe von feuchtigkeitsbefadenen Adsorbentien, beispielsweise von Molekularsieben und von wasserhaltigen, organischen Lösungsmitteln, beispielsweise von 80%-igem Ethanol erwiesen. Die Wasserzugabe kann aber auch über eine chemische Reaktion erfolgen, bei der Wasser im Laufe der Reaktion freigesetzt wird. Beispiele hierfür sind Veresterungen.

Wenn ein Lösungsmittel verwendet wird, kommen neben den niederen aliphatischen Alkoholen (beispielsweise Ethanol oder i-Propanol) auch Ketone, vorzugsweise niedere Dialkylketone, wie Aceton oder Methylisobutylketon, Ether, vorzugsweise niedere Dialkylether wie Diethylether oder Dibutylether, THF, Amide, Ester, insbesondere Essigsäureethylester, Dimethylformamid, Amine, insbesondere Triethylamin, und deren Gemische in Frage.

Werden Spiro-Silane zur Herstellung der Poly(hetero)kondensate eingesetzt, so wird die Hydrolyse bevorzugt in einem bzgl. der Spiro-Silane basischen Milieu durchgeführt. Dies wird entweder durch ein basisches Lösungsmittel, wie beispielsweise Triethylamin, oder durch Zugabe von basischen Hydrolyse- und Kondensationskatalysatoren, wie KOH, Methylimidazol erzeugt.

Die Ausgangsverbindungen müssen nicht notwendigerweise bereits alle zu Beginn der Hydrolyse (Polykondensation) vorhanden sein, sondern in bestimmten Fällen kann es sich sogar als vorteilhaft erweisen, wenn nur ein Teil dieser Verbindungen zunächst mit Wasser in Kontakt gebracht wird und später die restlichen Verbindungen zugegeben werden.

Um insbesondere bei Verwendung von von Siliciumverbindungen verschiedenen hydrolysierbaren Verbindungen Ausfällungen während der Hydrolyse und der Polykondensation so weit wie möglich zu vermeiden, kann die Wasserzugabe in mehreren Stufen, beispielsweise in drei Stufen, durchgeführt werden. Dabei kann in der ersten Stufe beispielsweise ein Zehntel bis ein Zwanzigstel der zur Hydrolyse benötigten Wassermenge zugegeben werden. Nach kurzem Rühren kann die Zugabe von einem Fünftel bis zu einem Zehntel der erforderlichen Wassermenge erfolgen, und nach weiterem kurzen Rühren kann schließlich der Rest zugegeben werden.

Die Kondensationszeit richtet sich nach den jeweiligen Ausgangskomponenten und deren Mengenanteilen, dem gegebenenfalls verwendeten Katalysator, der Reaktionstemperatur, etc. Im allgemeinen erfolgt die Polykondensation bei Normaldruck, sie kann jedoch auch bei erhöhtem oder bei verringertem Druck durchgeführt werden.

Das so erhaltene Poly(hetero)kondensat kann entweder als solches oder nach teilweiser oder nahezu vollständiger Entfernung des verwendeten Lösungsmittels weiterverarbeitet werden. In einigen Fällen kann es sich als vorteilhaft erweisen, in dem nach der Polykondensation erhaltenen Produkt das überschüssige Wasser und das gebildete und gegebenenfalls zusätzlich eingesetzte Lösungsmittel durch ein anderes Lösungsmittel zu ersetzen, um das Poly(hetero)kondensat zu stabilisieren. Zu diesem Zweck kann die Reaktionsmischung beispielsweise im Vakuum bei leicht erhöhter Temperatur so weit eingedickt werden, daß sie noch problemlos mit einem anderen Lösungsmittel aufgenommen werden kann.

Sollen diese Poly(hetero)kondensate als Lacke für die Beschichtung (beispielsweise von Kunststoffen wie PVC, PC, PMMA, PE, PS, von Glas, Papier, Holz, Keramik, Metall usw.) eingesetzt werden, so können diesen spätestens vor der Verwendung gegebenenfalls noch übliche Lackadditive zugegeben werden, wie Färbemittel (Pigmente oder Farbstoffe), Füllstoffe, Oxidationsinhibitoren, Flammschutzmittel, Verlaufsmittel, UV-Absorber, Stabilisatoren oder dergleichen. Auch Zusätze zur Erhöhung der Leitfähigkeit (beispielsweise Graphit-Pulver, Silber-Pulver) verdienen in diesem Zusammenhang Erwähnung. Im Falle der Verwendung als Formmasse kommt insbesondere die Zugabe von anorganischen und/oder organischen Füllstoffen in Frage, wie organische und anorganische Partikel, (Glas)Fasern, Mineralien.

Die endgültige Härtung der Poly(hetero)kondensate erfolgt nach Zugabe geeigneter Initiatoren thermisch, redoxinduziert, kovalent-nucleophil oder photochemisch, wobei auch mehrere Härtungsmechanismen parallel und/oder nacheinander ablaufen können. Dabei werden im Zuge der Polymerisation die C=C-Doppelbindungen verknüpft und es wird das organische Netzwerk aufgebaut. Aufgrund der relativ hohen Molekulargewichte der erfindungsgemäßen Silane erfahren diese bei der Härtung nur eine geringe Volumenschrumpfung.

Es ist auch möglich, dem Poly(hetero)kondensat vor der endgültigen Härtung, also vor der Polymerisation, weitere ionisch und/oder radikalisch polymerisierbare Komponenten zuzusetzen. Radikalisch polymerisierbare Verbindungen, die zugesetzt werden können, sind beispielsweise solche mit C=C-Doppelbindungen, wie etwa Acrylate oder Methacrylate, wobei die Polymerisation über die C=C-Doppelbindungen verläuft.

Ionisch polymerisierbare Verbindungen, die zugesetzt werden können, enthalten beispielsweise Ringsysteme, die kationisch, ringöffnend polymerisierbar sind, wie etwa Spiroorthoester, Spiroorthocarbonate, bicyclische Spiroorthoester, Mono- oder Oligoepoxide oder Spiro-Silane der allgemeinen Formel VIII. Es können aber auch Verbindungen zugesetzt werden, die sowohl kationisch als auch radikalisch polymerisierbar sind, wie beispielsweise Methacryloyl-Spiroorthoester. Diese sind radikalisch über die C=C-Doppelbindung und kationisch unter Ringöffnung polymerisierbar. Diese Systeme sind beispielsweise im Journal f. prakt. Chemie, Band 330, Heft 2, 1988, S. 316-318 oder im Journal of Polymer Science: Part C: Polymer Letters, Vol. 26, S. 517-520 (1988) beschrieben.

Erfolgt die Härtung des Poly(hetero)kondensates photochemisch, so werden diesem Photoinitiatoren zugesetzt, bei thermischer Härtung thermische Initiatoren und bei redox-induzierter Härtung Starter-Aktivator-Systeme.

Der Initiator kann in üblichen Mengen zugegeben werden. So kann beispielsweise einer Mischung, die 30 bis 50 Gew.-% Feststoff (Polykondensat) enthält, Initiatoren in einer Menge von beispielsweise 0,5 bis 5 Gew.-%, insbesondere von 1 bis 3 Gew.-%, bezogen auf die Mischung zugesetzt, werden.

Werden zur Herstellung der Poly(hetero)kondensate neben den erfindungsgemäßen Silanen weitere Komponenten eingesetzt, die reaktive Doppelbindungen enthalten, wie Silane gemäß der allgemeinen Formel VII, so kann über diese Doppelbindungen ebenfalls eine Polymerisation ablaufen, die thermisch und/oder photochemisch und/oder kovalent-nucleophil und/oder redox-initiiert sein kann.

Als Photoinitiatoren können beispielsweise die im Handel erhältlichen eingesetzt werden. Beispiele hierfür sind Irgacure 184 (1-Hydroxycyclohexylphenylketon), Irgacure 500 (1-Hydroxycyclohexylphenylketon-benzophenon), und andere von Ciba-Geigy erhältliche Photoinitiatoren vom Irgacure-Typ: Darocure 1173, 1116, 1398, 1174 und 1020 (erhältlich von der Firma Merck), Benzophenon, 2-Chlorthioxanthon, 2-Methylthioxanthon, 2-Isopropylthioxanthon, Benzoin, 4,4'-Dimethoxybenzoin etc. Erfolgt die Härtung mit sichtbarem Licht, wie beispielsweise im Dentalbereich, so kann als Initiator beispielsweise Campherchinon eingesetzt werden.

Als thermische Initiatoren kommen insbesondere organische Peroxide in Form von Diacylperoxiden, Peroxydicarbonaten, Alkylperestem, Dialkylperoxiden, Perketalen, Ketonperoxiden und Alkylhydroperoxiden in Frage. Konkrete und bevorzugte Beispiele für thermische Initiatoren sind Dibenzoylperoxid, t-Butylperbenzoat und Azobisisobutyronitril.

Als Starter-Aktivator-Systeme können hierfür übliche eingesetzt werden, wie beispielsweise aromatische Amine (beispielsweise N,N-Bis-(2-hydroxy-ethyl)-p-toluidin) als Aktivatoren oder als Starter beispielsweise Dibenzoylperoxid, wobei über deren Konzentration bzw. deren Konzentrationsverhältnis die Härtungszeit entsprechend dem jeweiligen Anwendungsfall eingestellt werden kann. Weitere Amine sind beispielsweise der DE-A-4 310 733 zu entnehmen.

Bei kovalent-nucleophiler Härtung werden als Initiatoren beispielsweise Verbindungen mit mindestens einer Aminogruppe zugesetzt. Geeignete Amine sind beispielsweise der DE-A-4 405 261 zu entnehmen.

Ein mit einem Initiator versehener Lack (Poly(hetero)kondensat) auf der Basis der erfindungsgemäßen Silane kann dann für Beschichtungen von Substraten eingesetzt werden. Für diese Beschichtung können übliche Beschichtungsverfahren angewendet werden, beispielsweise Tauchen, Fluten, Gießen, Schleudern, Walzen, Spritzen, Aufstreichen, elektrostatisches Spritzen und Elektrotauchlackierung. Erwähnt werden soll hier noch, daß der Lack nicht notwendigerweise lösungsmittelhaltig sein muß. Insbesondere bei Verwendung von Ausgangssubstanzen (Silanen) mit zwei Alkoxygruppen am Si-Atom kann auch ohne Zusatz von Lösungsmitteln gearbeitet werden.

Vor der Härtung läßt man vorzugsweise den aufgetragenen Lack abtrocknen. Danach kann er, abhängig von der Art des Initiators, redoxinduziert, thermisch oder photochemisch in an sich bekannter Weise gehärtet werden. Selbstverständlich sind auch Kombinationen von Aushärtungsmethoden möglich.

Erfolgt die Härtung des aufgetragenen Lackes durch Bestrahlen kann es sich von Vorteil erweisen, nach der Strahlungshärtung eine thermische Härtung durchzuführen, insbesondere um noch vorhandenes Lösungsmittel zu entfernen oder um noch weitere reaktive Gruppen in die Härtung miteinzubeziehen.

Obwohl in den Poly(hetero)kondensaten auf der Basis der erfindungsgemäßen Silane bereits polymerisierbare Gruppen vorhanden sind, kann es sich in bestimmten Fällen als vorteilhaft erweisen, diesen Kondensaten vor oder bei ihrer Weiterverarbeitung (Härtung) noch weitere Verbindungen (vorzugsweise rein organischer Natur) mit beispielsweise ungesättigten Gruppen zuzugeben.

Bevorzugte Beispiele für derartige Verbindungen sind Acrylsäure und Methacrylsäure sowie davon abgeleitete Verbindungen, insbesondere Ester von vorzugsweise einwertigen Alkoholen (beispielsweise $C_{1-4}$-Alkanolen), (Meth)Acrylnitril, Styrol und Mischungen derselben. Im Fall der Verwendung der Poly(hetero)kondensate zur Herstellung von Beschichtungslacken können derartige Verbindungen gleichzeitig als Lösungs- bzw. Verdünnungsmittel wirken.

Die Herstellung von Formkörpern bzw. Formmassen aus Poly(hetero)kondensaten auf der Basis der erfindungsgemäßen Silane kann mit jeder auf diesem Gebiet gebräuchlichen Methode erfolgen, beispielsweise durch Spritzguß, Formgießen, Extrusion. Auch zur Herstellung von Kompositmaterialien (beispielsweise mit Glasfaserverstärkung) sind die Poly(hetero)kondensate auf der Basis der erfindungsgemäßen Silane geeignet.

Mit den erfindungsgemäßen, mehrfach funktionellen Silanen stehen Ausgangsverbindungen zur Verfügung, die die Herstellung von anorganisch-organischen Verbundpolymeren mit den unterschiedlichsten und in weiten Bereichen einstellbaren Eigenschaften bzw. die Modifikation von bestehenden Verbundpolymeren ermöglichen. Der Einsatz solcher Materialien erstreckt sich auf die verschiedensten Zwecke und unter anderem auf die Verwendung als Bulkmaterialien, Komposite, Klebstoffe, Vergußmassen, Beschichtungsmaterialien, Haftvermittler und Bindemittel für keramische Partikel (keramische Formgebungsverfahren), zur Herstellung bzw. Primung von Füllstoffen und Fasern, von Schleifscheiben, als Einsatz im Reaktionsextruder etc.. Für die organische Polymerisation kommt die photochemisch, die thermisch sowie die chemisch (2-Komponenten, anaerob, Redox etc.) induzierte Umsetzung in Frage. Die Kombination von Selbsthärtung mit beispielsweise photoinduzierter bzw. thermischer Härtung ist ebenfalls möglich.

Die Erfindung wird nachfolgend durch Beispiele näher erläutert, ohne daß sie durch diese beschränkt werden soll.

**Herstellungsbeispiel 1**

Darstellung des Hydrosilylierungsproduktes von 1-(Trimethoxysilylethyl)-1,1,3,3-tetramethyldisiloxan mit Allyloxyethylmethacrylat

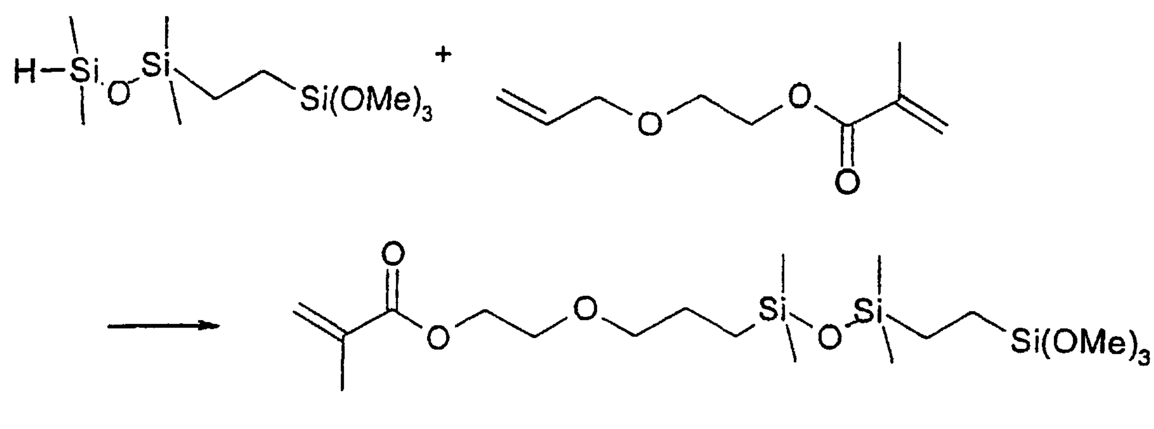

1-(Trimethoxysilylethyl)-1,1,3,3-tetramethyldisiloxan wurde nach J.V. Crivello und Daoshen Bi (J. Polym. Sci

A 31 (1993) 3121) aus 1,1,3,3-Tetramethyldisiloxan und Vinyltrimethoxysilan erhalten.

14,1 g (0,05 M) 1-(Trimethoxysilylethyl)-1,1,3,3-tetramethyldisiloxan werden in 100 ml trockenem Toluol unter Inertgas mit 5 mg polymer-gebundenem Wilkinson Katalysator und 8,5 g (0,05 M) Allyloxyethylmethacrylat auf 100 °C erhitzt. Nach vier Stunden sind die Allyl- und SiH-Absorptionen im $^1$H-NMR-Spektrum verschwunden. Man läßt abkühlen, filtriert vom Katalysator und zieht das Lösungsmittel unter vermindertem Druck ab.

Es verbleibt ein farbloses, niedrigviskoses Öl. Die Ausbeute beträgt 98 % der Theorie.

**Herstellungsbeispiel 2**

Hydrolyse und Kondensation des Produktes aus Herstellungsbeispiel 1

9,1 g (0,02 Mol) der Verbindung aus Herstellungsbeispiel 1 werden mit 1,1 g (0,06 Mol) Wasser (zugesetzt als 0,1 M HCl) in 50 ml Essigester 24 h bei 40 °C gerührt. Danach wird mit $NaHCO_3$-Lösung neutralisiert, getrocknet und von Lösungsmittel befreit. Das Kondensat ist fließfähig und wird in einer Ausbeute von 96 % der Theorie erhalten.

**Herstellungsbeispiel 3**

Co-Hydrolyse des Produktes aus Beispiel 1 mit TMOS

9,1 g (0,02 Mol) der Verbindung aus Herstellungsbeispiel 1 werden mit 0,76 g (0,005 Mol) Tetramethylorthosilikat (TMOS) versetzt und in 50 ml Essigester mit 1,4 g (0,08 Mol) Wasser (zugesetzt als 0,1 M HCl) 36 Stunden bei 36 °C gerührt. Die Lösung wird mittels $NaHCO_3$-Lösung neutralisiert, getrocknet und von Lösungsmittel befreit. Die Ausbeute an viskosem Kondensat beträgt 95 % der Theorie.

**Herstellungsbeispiel 4**

Darstellung des Hydrosilylierungsproduktes aus 1-(Trimethoxysilylethyl)-1,1,4,4-tetramethyldisilabutan und Glycerindimethacrylat-allylether

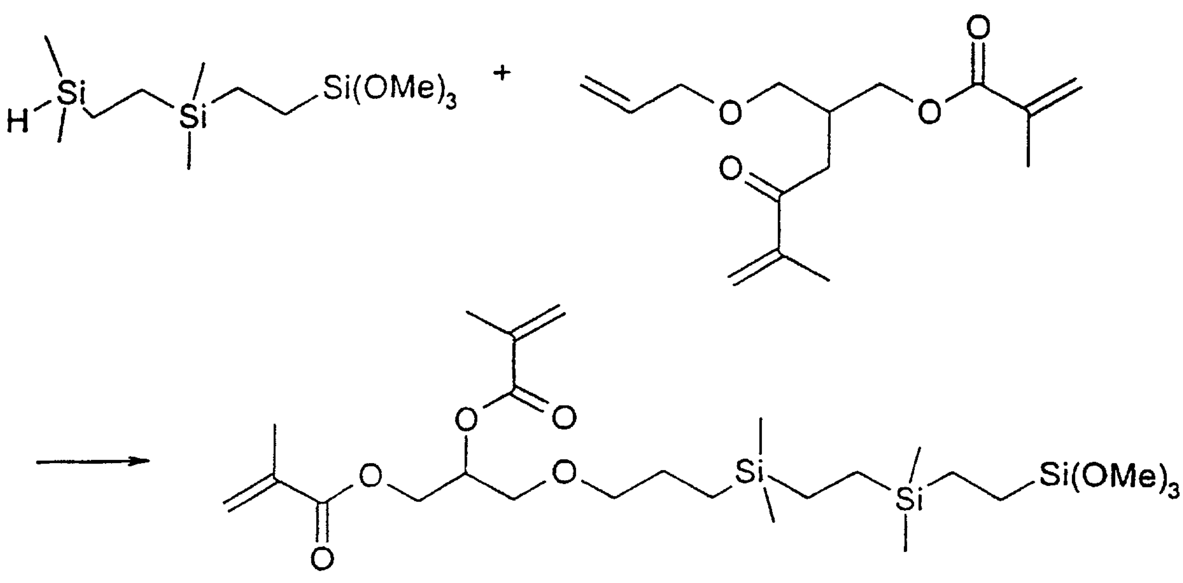

1-(Trimethoxysilylethyl)-1,1,4,4-tetramethyldisilabutan wird analog 1-(Trimethoxysilylethyl)-1,1,3,3-tetramethyldisiloxan in Herstellungsbeispiel 1 aus 1,2-Bisdimethylsilylethan hergestellt. Es wird als farbloses, hydrolyselabiles Öl in einer Ausbeute von 54 % der Theorie erhalten.

5,9 g (0,02 M) 1-(Trimethoxysilylethyl)-1,1,4,4-tetramethyldisilabutan werden in 100 ml trockenem Toluol gelöst und mit 5 mg Deloxan Pt-Katalysator und bei Rückfluß mit 5,4 g (0,02 M) Glycerindimethacrylat-allylether versetzt. Nach Versiegen der Wärmetönung wird eine Probe gezogen und mittels IR auf die SiH-Schwingung bei 2160 $cm^{-1}$

geprüft. Ist diese verschwunden, wird vom Katalysator abfiltriert und das Lösungsmittel abdestilliert. Das Addukt wird als farbloses, hydrolyselabiles Öl in einer Ausbeute von 94 % der Theorie erhalten.

**Herstellungsbeispiel 5**

Hydrolyse und Kondensation des Produktes aus Herstellungsbeispiel 4

11,3 g (0,02 Mol) der Verbindung aus Beispiel 4 werden mit 1,1 g (0,06 Mol) Wasser (zugesetzt als 0,1 M HCl) in 50 ml Essigester 24 h bei 40 °C gerührt. Danach wird mit $NaHCO_3$-Lösung neutralisiert, getrocknet und von Lösungsmittel befreit. Das Kondensat ist fließfähig und wird in einer Ausbeute von 97 % der Theorie erhalten.

**Herstellungsbeispiel 6**

Co-Hydrolyse des Produktes aus Herstellungsbeispiel 4 mit TMOS

11,3 g (0,02 Mol) der Verbindung aus Beispiel 4 werden mit 0,8 g (0,005 Mol) Tetramethylorthosilikat versetzt und in 50 ml Essigester mit 1,4 g (0,08 Mol) Wasser (zugesetzt als 0,1 M HCl) 36 Stunden bei 36 °C gerührt. Die Lösung wird mittels $NaHCO_3$-Lösung neutralisiert, getrocknet und von Lösungsmittel befreit. Die Ausbeute an viskosem Kondensat beträgt 96 % der Theorie.

**Herstellungsbeispiel 7**

Darstellung des Hydrosilylierungsproduktes aus 1-(Trimethoxysilylethyl)-1-methyl-1-phenylsilan und Glycerindimethacrylat-allylether

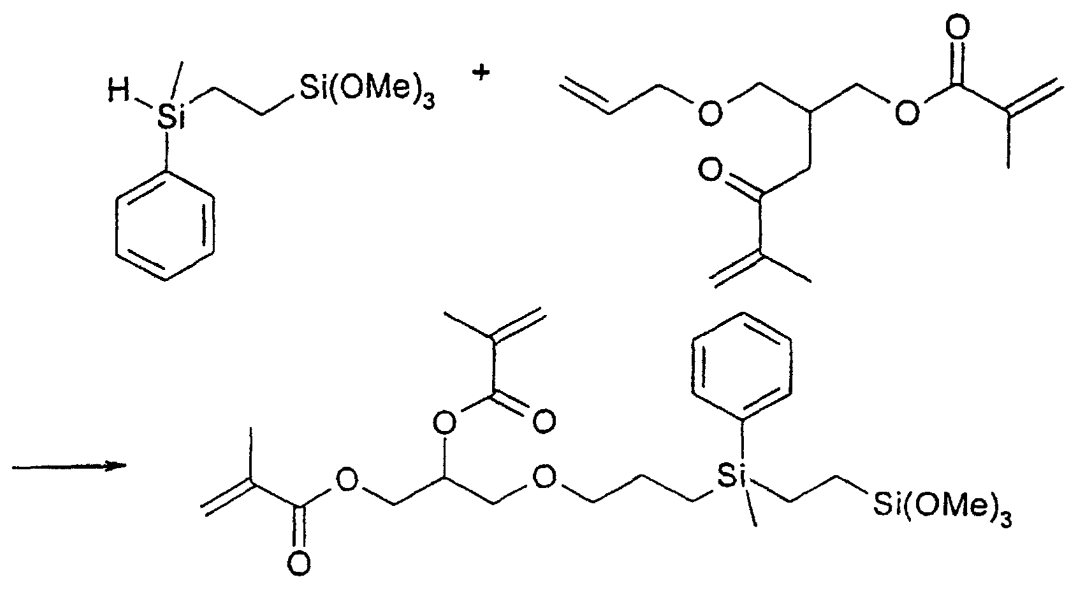

1-(Trimethoxysilylethyl)-1-methyl-1-phenylsilan wird analog 1-(Trimethoxysilylethyl)-1,1,3,3-tetramethyldisiloxan in Herstellungsbeispiel 1 aus 1-Methyl-1-phenylsilan hergestellt. Es wird als farbloses, hydrolyselabiles Öl in einer Ausbeute von 69 % der Theorie erhalten.

13,5 g (0,05 Mol) 1-(Trimethoxysilylethyl)-1-methyl-1-phenylsilan werden in 100 ml trockenem Toluol gelöst und mit 5 mg Deloxan Pt-Katalysator und bei Rückfluß mit 13,4 g (0,05 Mol) Glycerindimethacrylat-allylether versetzt. Nach Versiegen der Wärmetönung wird eine Probe gezogen und mittels IR auf die SiH-Schwingung bei 2160 $cm^{-1}$ geprüft. Ist diese verschwunden wird vom Katalysator abfiltriert und das Lösungsmittel abdestilliert. Das Addukt wird als farbloses, hydrolyselabiles Öl in einer Ausbeute von 94 % der Theorie erhalten.

**Herstellungsbeispiel 8**

Hydrolyse und Kondensation des Produktes aus Herstellungsbeispiel 7

10,8 g (0,02 Mol) der Verbindung aus Herstellungsbeispiel 7 werden mit 1,1 g (0,06 Mol) Wasser (zugesetzt als 0,1 M HCl) in 50 ml Essigester 24 h bei 40 °C gerührt. Danach wird mit $NaHCO_3$-Lösung neutralisiert, getrocknet und von Lösungsmittel befreit. Das Kondensat ist fließfähig und wird in einer Ausbeute von 97 % der Theorie erhalten.

**Herstellungsbeispiel 9**

Co-Hydrolyse des Produktes aus Herstellungsbeispiel 7 mit TMOS

10,8 g (0,02 Mol) der Verbindung aus Herstellungsbeispiel 7 werden mit 0,8 g (0,005 Mol) Tetramethylorthosilikat versetzt und in 50 ml Essigester mit 1,4 g (0,08 Mol) Wasser (zugesetzt als 0,1 M HCl) 36 Stunden bei 36 °C gerührt. Die Lösung wird mittels $NaHCO_3$-Lösung neutralisiert, getrocknet und von Lösungsmittel befreit. Die Ausbeute an viskosem Kondensat beträgt 96 % der Theorie.

**Herstellungsbeispiel 10**

Darstellung des Hydrosilylierungsproduktes von 1-(Trimethoxysilylethyl)-1,1,3,3-tetramethyldisiloxan mit Vinylnorbornen

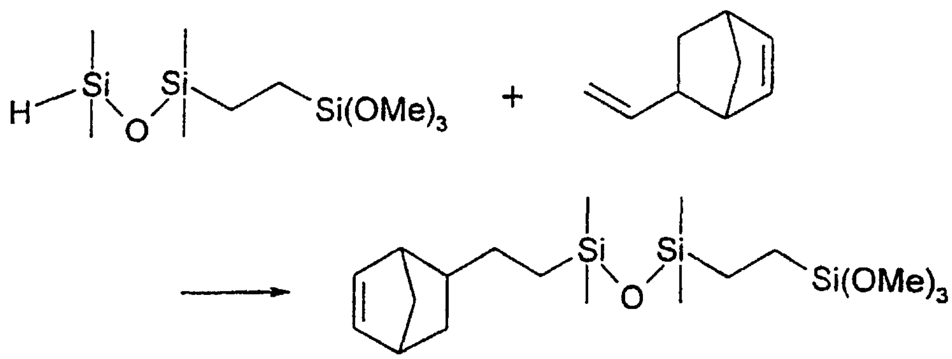

1-(Trimethoxysilylethyl)-1,1,3,3-tetramethyldisiloxan wurde nach J.V. Crivello und Daoshen Bi (J. Polym. Sci A 31 (1993) 3121) aus 1,1,3,3-Tetramethyldisiloxan und Vinyltrimethoxysilan erhalten.

14,1 g (0,05 Mol) 1-(Trimethoxysilylethyl)-1,1,3,3-tetramethyldisiloxan werden in 100 ml trockenem Toluol unter Inertgas mit 5 mg polymer-gebundenem Wilkinson Katalysator und 6,0 g (0,05 Mol) Vinylnorbornen auf 100 °C erhitzt. Nach vier Stunden sind die Vinyl-Absorptionen im $^{1}H$-NMR-Spektrum verschwunden. Man läßt abkühlen, filtriert vom Katalysator und zieht das Lösungsmittel unter vermindertem Druck ab.

Es verbleibt ein farbloses, niedrigviskoses Öl. Die Ausbeute beträgt 98 % der Theorie.

**Herstellungsbeispiel 11**

Hydrolyse und Kondensation des Produktes aus Herstellungsbeispiel 10

8,1 g (0,02 Mol) der Verbindung aus Herstellungsbeispiel 10 werden mit 1,1 g (0,06 Mol) Wasser (zugesetzt als 0,1 M HCl) in 50 ml Essigester 24 h bei 40 °C gerührt. Danach wird mit $NaHCO_3$-Lösung neutralisiert, getrocknet und von Lösungsmittel befreit. Das Kondensat ist fließfähig und wird in einer Ausbeute von 96 % der Theorie erhalten.

**Herstellungsbeispiel 12**

Co-Hydrolyse des Produktes aus Herstellungsbeispiel 10 mit TMOS

8,1 g (0,02 Mol) der Verbindung aus Herstelungsbeispiel 10 werden mit 0,8 g (0,005 Mol) Tetramethylorthosilikat versetzt und in 50 ml Essigester mit 1,4 g (0,08 Mol) Wasser (zugesetzt als 0,1 M HCl) 36 Stunden bei 36 °C gerührt. Die Lösung wird mittels $NaHCO_3$-Lösung neutralisiert, getrocknet und von Lösungsmittel befreit. Die Ausbeute an viskosem Kondensat beträgt 95 % der Theorie.

**Herstellungsbeispiel 13**

Darstellung des Hydrosilylierungsproduktes von 1-(Methyldimethoxysilylethyl)-1,1,3,3-tetramethyldisiloxan mit Trimethylolpropantriacrylat

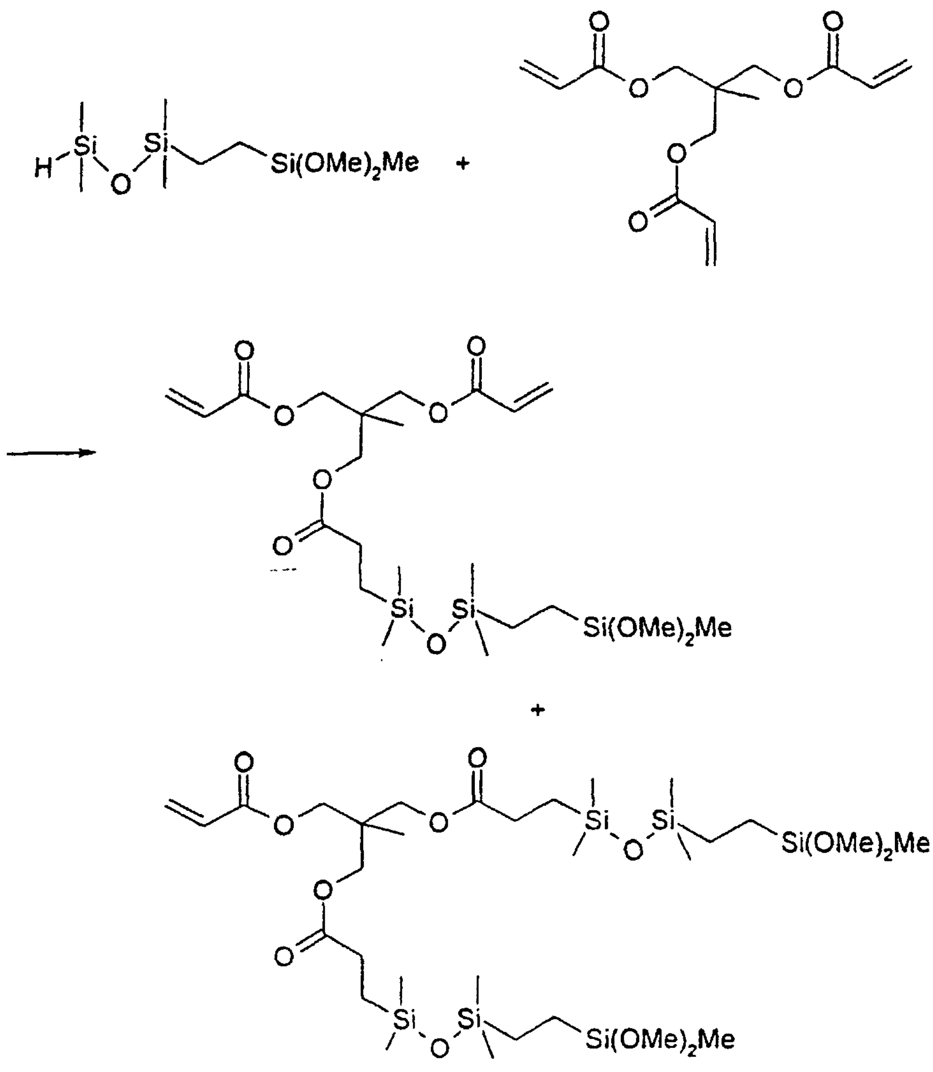

1-(Methyldimethoxysilylethyl)-1,1,3,3-tetramethyldisiloxan wurde analog J.V. Crivello und Daoshen Bi (J. Polym. Sci A 31 (1993) 3121) aus 1,1,3,3-Tetramethyldisiloxan und Vinylmethyldimethoxysilan in einer Ausbeute von 67 % der Theorie erhalten.

8,0 g (0,03 Mol) 1-(Methyldimethoxysilylethyl)-1,1,3,3-tetramethyldisiloxan werden in 100 ml trockenem Toluol unter Inertgas mit 5 mg polymer-gebundenem Wilkinson Katalysator sowie 14,4 g (0,05 Mol) Trimethylolpropantriacrylat auf 100 °C erhitzt. Nach vier Stunden sind die Vinyl-Absorptionen im $^1H$-NMR-Spektrum verschwunden.

Man läßt abkühlen, filtriert vom Katalysator und zieht das Lösungsmittel unter vermindertem Druck ab.

Es verbleibt ein farbloses, niedrigviskoses Öl. Die Ausbeute beträgt 98 % der Theorie.

**Herstellungsbeispiel 14**

Hydrolyse und Kondensation des Produktes aus Herstellungsbeispiel 13

7,5 g (0,01 Mol, bezogen auf den Dimethoxysilyl-Gehalt) der Verbindung aus Herstellungsbeispiel 13 werden mit 0,4 g (0,02 Mol) Wasser (zugesetzt als 0,1 M HCl) in 50 ml Essigester 24 h bei 40 °C gerührt. Danach wird mit $NaHCO_3$-Lösung neutralisiert, getrocknet und von Lösungsmittel befreit. Das Kondensat ist fließfähig und wird in einer Ausbeute von 96 % der Theorie erhalten.

**Herstellungsbeispiel 15**

Co-Hydrolyse des Produktes aus Herstellungsbeispiel 13 mit TMOS

7,5 g (0,01 Mol bezogen auf Dimethoxysilyl-Gehalt) der Verbindung aus Herstellungsbeispiel 13 werden mit 0,005 Mol Tetramethylorthosilikat versetzt und in 50 ml Essigester mit 0,7 g (0,04 Mol) Wasser (zugesetzt als 0,1 M HCl) 36 Stunden bei 36 °C gerührt. Die Lösung wird mittels $NaHCO_3$-Lösung neutralisiert, getrocknet und von Lösungsmittel befreit. Die Ausbeute an viskosem Kondensat beträgt 95 % der Theorie.

**Patentansprüche**

**1.** Hydrolysierbare und polymerisierbare Silane der allgemeinen Formel I

$$B\text{-}R'\text{-}U\text{-}D \qquad (I)$$

in der die Reste folgende Bedeutung haben:

B = Vinylcyclopropyl,

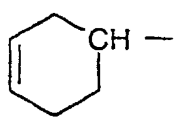
,
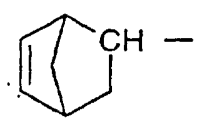

oder Methacrylatreste (einschließlich der entsprechenden Acrylatreste) aus der folgenden Gruppe:

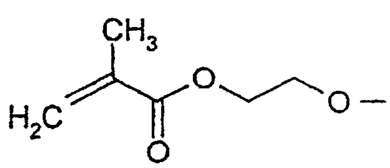

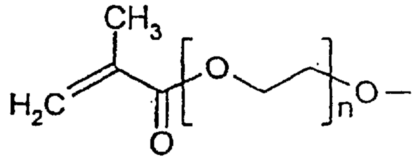

n = 2, 3, 4, 5, 6, 7, bzw. Gemische mit mittlerem n < 10

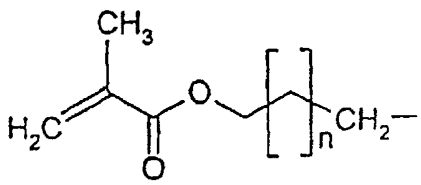

n = 0, 1, 2, 3, 4, 5, 6, 7, 8, 9, 10

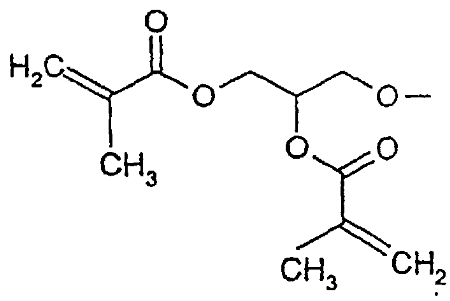

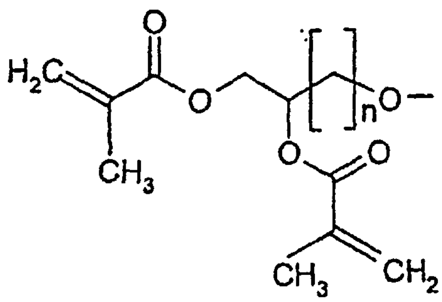

n = 1, 2, 3, 4, 5, 6, 7, 8, 9, 10

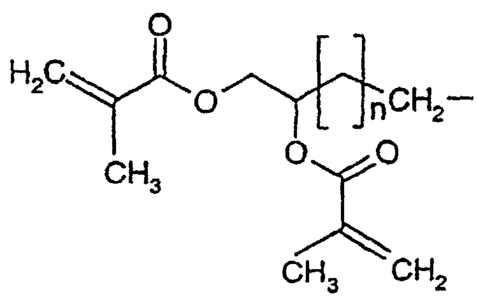

n = 0, 1, 2, 3, 4, 5, 6, 7, 8, 9, 10

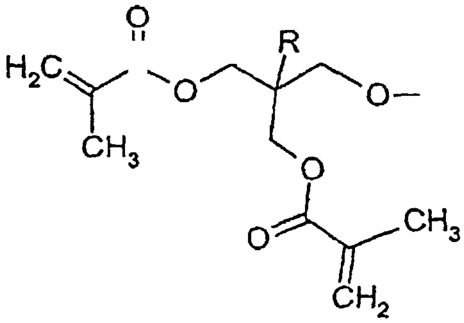

R = Alkyl, Aryl, Alkylaryl

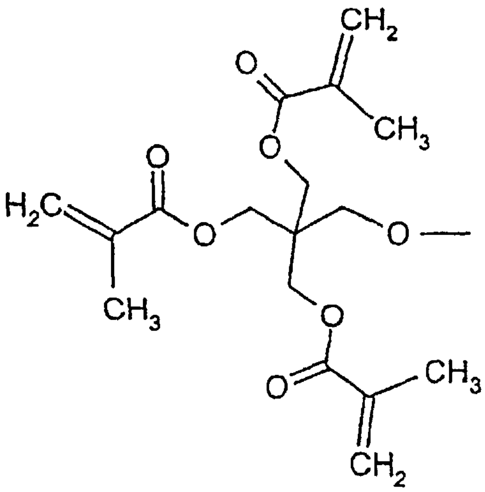

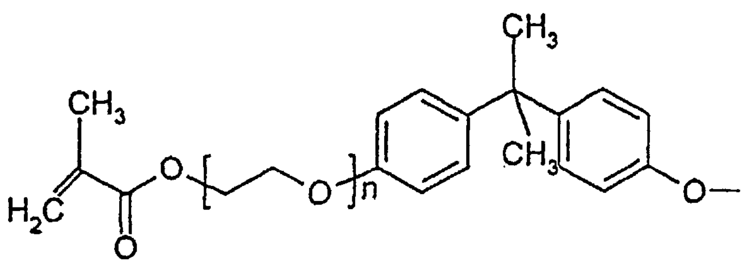

n = 1, 2, 3, 4, 5

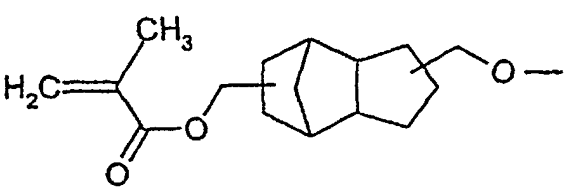
,
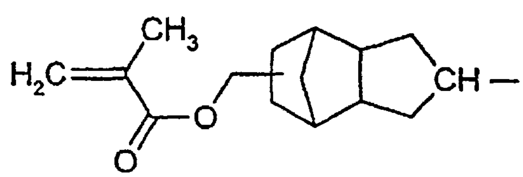
;

R' = Alkylen, Alkenylen, Arylen, Arylenalkylen oder Alkylenarylen mit jeweils 0 bis 10 Kohlenstoffatomen, wobei diese Reste durch Sauerstoff- und Schwefelatome oder durch Aminogruppen unterbrochen sein können;

U =

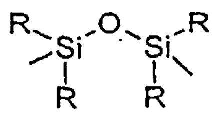

R = Alkyl, Aryl, Cycloyalkyl

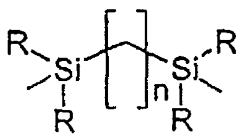

R = Alkyl, Aryl, Cycloyalkyl n = 1, 2, 3, 4, 5, 6, 7, 8, 9, 10

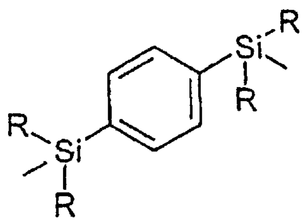

R = Alkyl, Aryl, Cycloyalkyl

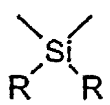

R = Alkyl, Aryl, Cycloalkyl

D = $-(CH_2)_n-Si(CH_3)_2(OC_2H_5)$, mit n = 0 bis 10
$-(CH_2)_n-Si(CH_3)(OC_2H_5)_2$, mit n = 0 bis 10
$-(CH_2)_n-Si(OCH_3)_3$, mit n = 0 bis 10
$-(CH_2)_n-Si(C_2H_5)_2(OCH_3)$, mit n = 0 bis 10
$-(CH_2)_n-Si(C_2H_5)(OCH_3)_2$, mit n = 0 bis 10
$-(CH_2)_n-Si(OC_2H_5)_3$, mit n = 0 bis 10
$-(CH_2)_n-Si(CH_3)_2(OCH_3)$, mit n = 0 bis 10
$-(CH_2)_n-Si(CH_3)(OCH_3)_2$, mit n = 0 bis 10
$-(CH_2)_n-Si(C_2H_5)_2(OC_2H_5)$, mit n = 0 bis 10
$-(CH_2)_n-Si(C_2H_5)(OC_2H_5)_2$, mit n = 0 bis 10
$-(CH_2)_n-Si(CH_3)(C_2H_5)(OCH_3)$, mit n = 0 bis 10
$-(CH_2)_n-Si(CH_3)(C_2H_5)(OC_2H_5)$, mit n = 0 bis 10
$-(CH_2)_n-Si(CH_3)(OC_2H_5)(OCH_3)$, mit n = 0 bis 10
$-(CH_2)_n-Si(C_2H_5)(OC_2H_5)(OCH_3)$, mit n = 0 bis 10
$-(CH_2)_n-Si(OC_2H_5)_2(OCH_3)$, mit n = 0 bis 10
$-(CH_2)_n-Si(OC_2H_5)(OCH_3)_2$, mit n = 0 bis 10
$-(CH_2)_n-Si(CH_3)(OC(CH_3)=CH_2)_2$, mit n = 0 bis 10
$-(CH_2)_n-Si(C_2H_5)(OC(CH_3)=CH_2)_2$, mit n = 0 bis 10
$-(CH_2)_n-Si(CH_3)_2(OC(CH_3)=CH_2)$, mit n = 0 bis 10
$-(CH_2)_n-Si(C_2H_5)_2(OC(CH_3)=CH_2)$, mit n = 0 bis 10
$-(CH_2)_n-Si(OCH_3)(OC(CH_3)=CH_2)_2$, mit n = 0 bis 10
$-(CH_2)_n-Si(OC_2H_5)(OC(CH_3)=CH_2)_2$, mit n = 0 bis 10
$-(CH_2)_n-Si(OCH_3)_2(OC(CH_3)=CH_2)$, mit n = 0 bis 10

$-(CH_2)_n-Si(OC_2H_5)_2(OC(CH_3)=CH_2)$, mit n = 0 bis 10
$-(CH_2)_n-Si(C_6H_5)(OC_2H_5)_2$, mit n = 0 bis 10
$-(CH_2)_n-Si(C_6H_5)(OCH_3)_2$, mit n = 0 bis 10
$-(CH_2)_n-Si(C_6H_5)(OCH_3)(OC_2H_5)$, mit n = 0 bis 10
$-(CH_2)_n-Si(C_6H_5)(OC(CH_3)=CH_2)_2$, mit n = 0 bis 10.

**2.** Silane nach Anspruch 1, **dadurch gekennzeichnet, daß** in der allgemeinen Formel der Rest R' die folgende Bedeutung hat:

R' = Alkylen, Alkenylen, Arylen, Arylenalkylen oder Alkylenarylen mit jeweils 0 bis 5 Kohlenstoffatomen, wobei diese Reste durch Sauerstoff- und Schwefelatome oder durch Aminogruppen unterbrochen sein können.

**3.** Verfahren zur Herstellung der Silane nach Anspruch 1 oder 2, **dadurch gekennzeichnet, daß** eine Dihydridosiliciumverbindung mit einer Alkenylsilanverbindung, die hydrolysierbare Gruppen enthält, äquimolar umgesetzt wird und die verbliebene Hydridosilylgruppe durch erneute Hydrosilylierung mit einer mindestens zweifach ungesättigten organischen Verbindung umgesetzt wird.

**4.** Verwendung der Silane nach Anspruch 1 oder 2 zur Herstellung von Kieselsäurepolykondensaten oder von Kieselsäureheteropolykondensaten durch hydrolytische Kondensation von einer oder mehreren hydrolytisch kondensierbaren Verbindungen des Siliciums und gegebenenfalls anderer Elemente aus der Gruppe B, Al, P, Sn, Pb, der Übergangsmetalle, der Lanthaniden und der Actiniden, und/oder von den oben genannten Verbindungen abgeleiteten Vorkondensaten, gegebenenfalls in Anwesenheit eines Katalysators und/oder eines Lösungsmittels, durch Einwirkung von Wasser oder von Feuchtigkeit, **dadurch gekennzeichnet, daß** 5 bis 100 Mol-% auf der Basis monomerer Verbindungen der hydrolytisch kondensierbaren Verbindungen aus Silanen der allgemeinen Formel I ausgewählt werden:

$$B\text{-}R'\text{-}U\text{-}D \qquad (I)$$

in der die Reste wie in Anspruch 1 definiert sind.

**5.** Verwendung nach Anspruch 4, **dadurch gekennzeichnet, daß** man als weitere hydrolytisch kondensierbare Verbindungen solche Verbindungen einsetzt, gegebenenfalls in vorkondensierter Form, die radikalisch oder ionisch polymerisierbar sind.

**6.** Verwendung nach Anspruch 4 oder 5, dadurch gekenneichnet, daß man als weitere hydrolytisch kondensierbare Verbindungen des Siliciums eine oder mehrere Verbindungen der allgemeinen Formel VI, gegebenenfalls in vorkondensierter Form, einsetzt:

$$R_e(R^{10}Z')_fSiX_{4-(e+f)} \qquad (IV)$$

in der die Reste und Indices die folgende Bedeutung haben:

X = Wasserstoff, Halogen, Hydroxy, Alkoxy, Acyloxy, Alkylcarbonyl, Alkoxycarbonyl oder $NR''_2$;
R= Alkyl, Alkenyl, Aryl, Alkylaryl oder Arylalkyl;
R" = Wasserstoff, Alkyl oder Aryl;
$R^{10}$ = Alkylen oder Alkenylen, wobei diese Reste durch Sauerstoff- oder Schwefelatome oder -NH-Gruppen unterbrochen sein können;
Z' = Halogen oder eine gegebenenfalls substituierte Amino-, Amid-, Aldehyd-, Alkylcarbonyl-, Carboxy-, Mercapto-, Cyano-, Alkoxy-, Alkoxycarbonyl-, Sulfonsäure-, Phosphorsäure-, Acryloxy-, Methacryloxy-, Epoxy- oder Vinylgruppe;
e= 0, 1, 2 oder 3;
f= 0, 1, 2 oder 3, mit e + f = 1, 2 oder 3.

**7.** Verwendung nach einem der Ansprüche 4 bis 6, **dadurch gekennzeichnet, daß** man als weitere hydrolytisch kondensierbare Verbindungen des Siliciums eine oder mehrere Verbindungen der allgemeinen Formel VIII, gegebenenfalls in vorkondensierter Form einsetzt:

$$Y_n \, Si \, X_m \, R_{4-(n+m)} \quad \text{(VIII)}$$

in der die Reste X und R die in Anspruch 6 angegebene Bedeutung und die weiteren Reste und Indices die folgende Bedeutung haben:

Y = ein Substituent, der einen substituierten oder unsubstituierten 1,4,6-Trioxaspiro-[4,4]-nonanrest enthält;
n = 1, 2 oder 3;
m = 1, 2 oder 3, mit $n + m \leqq 4$.

**8.** Verwendung nach einem der Ansprüche 4 bis 7, **dadurch gekennzeichnet, daß** man als weitere hydrolytisch kondensierbare Komponente eine oder mehrere, im Reaktionsmedium lösliche Aluminium-, Titan- oder Zirkoniumverbindungen, gegebenenfalls in vorkondensierter Form, der Formel:

$$AlR^0 \text{ oder } M \, X_y \, R_z$$

einsetzt, in der M Titan oder Zirkonium bedeutet, die Reste R, R° und X gleich oder verschieden sind, $R^0$ Halogen, Hydroxy, Alkoxy oder Acyloxy darstellt, y eine ganze Zahl von 1 bis 4 ist, insbesondere 2 bis 4, z für 0, 1, 2 oder 3 steht, vorzugsweise für 0, 1 oder 2 und X und R wie in Anspruch 6 definiert sind.

**9.** Verwendung nach einem oder mehreren der Ansprüche 4 bis 8, **dadurch gekennzeichnet, daß** man dem Polykondensat einen oder mehrere Initiatoren zusetzt und das Polykondensat thermisch, photochemisch, kovalent-nucleophil oder redoxinduziert härtet.

**10.** Verwendung nach einem oder mehreren der Ansprüche 4 bis 8, **dadurch gekennzeichnet, daß** man dem Polykondensat vor der Polymerisation eine oder mehrere radikalisch undloder ionisch polymerisierbare Komponenten zusetzt.

**11.** Verwendung der Silane nach Anspruch 1 zur Herstellung von Polymerisaten durch radikalische Polymerisation einer oder mehrere C=C-Doppelbindungen enthaltender Verbindungen und gegebenenfalls anderer radikalisch polymerisierbarer Verbindungen und gegebenenfalls durch ionische Polymerisation einer oder mehrerer ionisch polymerisierbarer Verbindungen, durch Einwirkung von Wärme und/oder elektromagnetischer Strahlung undloder redoxinduziert und/oder kovalent-nucleophil, gegebenenfalls in Anwesenheit eines oder mehrere Inititatoren und/oder eines Lösungsmittels, **dadurch gekennzeichnet, daß** 5 bis 100 Mol-%, auf der Basis monomerer Verbindungen, aus Silanen der Formel I:

$$B\text{-}R'\text{-}U\text{-}D \quad \text{(I)}$$

ausgewählt werden, in der die Reste wie in Anspruch 1 oder 2 definiert sind.

**12.** Verwendung nach Anspruch 11, **dadurch gekennzeichnet, daß** man als kationisch polymerisierbare Verbindungen eines oder mehrere Silane der allgemeinen Formel VIII einsetzt:

$$Y_n \, Si \, X_m \, R_{4-(n+m)} \quad \text{(III)}$$

in der die Reste und Indices wie in Anspruch 7 definiert sind

**13.** Verwendung nach einem oder mehreren der Ansprüche 11 bis 12, **dadurch gekennzeichnet, daß** man das Polymerisat, gegebenenfalls in Anwesenheit weiterer, hydrolytisch kondensierbarer Verbindungen des Siliciums und gegebenenfalls anderer Elemente aus der Gruppe B, Al, Sn, Pb, der Übergangsmetalle, der Lanthaniden und der Actiniden, und/oder von den oben genannten Verbindungen abgeleiteten Vorkondensaten, durch Einwirkung von Wasser oder Feuchtigkeit, gegebenenfalls in Anwesenheit eines Katalysators und/oder eines Lösungsmittels, hydrolytisch kondensiert.

**14.** Verwendung nach Anspruch 13, **dadurch gekennzeichnet, daß** man als weitere kondensierbare Verbindungen des Siliciums eine oder mehrere Verbindungen der allgemeinen Formel VI, gegebenenfalls in vorkondensierter Form, einsetzt:

$$R_e(R^{10}Z')_fSiX_{4-(e+f)} \qquad (VI)$$

in der die Reste und Indices wie in Anspruch 6 definiert sind.

**15.** Verwendung nach einem der Ansprüche 4 bis 10, **dadurch gekennzeichnet, daß** man als weitere kondensierbare Verbindungen des Siliciums eine oder mehrere Verbindungen der allgemeinen Formel IX, gegebenenfalls in vorkondensierter Form, einsetzt:

$$G\{A-(Z)_d-R^{20}(R^{21})-R'-SiX_aR_b\}_c \qquad (IX)$$

in der die Reste und Indices die folgende Bedeutung haben:

X = Wasserstoff, Halogen, Hydroxy, Alkoxy, Acyloxy, Alkylcarbonyl, Alkoxycarbonyl oder $NR''_2$;
R = Alkyl, Alkenyl, Aryl, Alkylaryl oder Arylalkyl;
R' = Alkylen, Alkenylen, Arylen, Arylenalkylen oder Alkylenarylen mit jeweils 0 bis 10 Kohlenstoffatomen, wobei diese Reste durch Sauerstoff- und Schwefelatome oder durch Aminogruppen unterbrochen sein können;
R" = Wasserstoff, Alkyl oder Aryl;
G = ein geradkettiger oder verzweigter organischer Rest mit mindestens einer C=C-Doppelbindung und 4 bis 50 Kohlenstoffatomen;
A = O, S oder NH für d = 1 und

Z = CO und
$R^{20}$ = Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und
$R^{21}$= COOH;

oder

A = O, S oder NH für d = 1 und

Z = CO und
$R^{20}$= Alkylen, Arylen oder Alkylenarylen mit jeweils 1 bis 10 Kohlenstoffatomen, wobei diese Rest durch Sauerstoff- und Schwefelatome oder durch Aminogruppenunterbrochen sein können, und
$R^{21}$= H;

oder

A = O, S, NH oder COO für d = 1 und

Z = CHR, mit R gleich H, Alkyl, Aryl oder Alkylaryl, und
$R^{20}$= Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und
$R^{21}$ = OH;

oder

A = O, S, NH oder COO für d = 0 und

$R^{20}$= Alkylen, Arylen oder Alkylenarylen mit jeweils 1 bis 10 Kohlenstoffatomen, wobei diese Reste

durch Sauerstoff- und Schwefelatome oder durch Aminogruppen unterbrochen sein können, und

$R^{21}$ = OH;

oder

A = S für d = 1 und

Z = CO und
$R^{20}$= N und
$R^{21}$= H;

a= 1, 2 oder 3;
b= 0, 1 oder 2;
a + b = 3;
c= 1, 2, 3 oder 4;
d= 0 oder 1.

**16.** Verwendung der Silane nach Anspruch 1 oder 2 im dentalen Bereich zur Herstellung von Füllungsmaterialien, Zementen, provisorischen Kronen- und Brückenmaterialien, Verblendmaterialien, Lacken, Sealern, Haftvermittlern, Primern und Bondings.

**Claims**

**1.** Hydrolyzable and polymerizable silanes of the general formula I

$$B\text{-}R'\text{-}U\text{-}D \qquad (I)$$

in which the radicals have the following meaning:

B = vinylcyclopropyl,

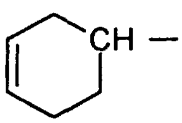 , 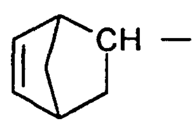

or methacrylate radicals (including the corresponding acrylate radicals) from the following group:

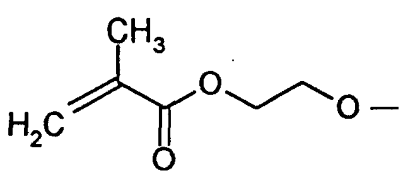

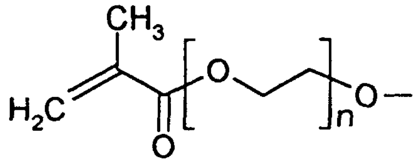

n=2,3,4,5,6,7, or mixtures with average n<10

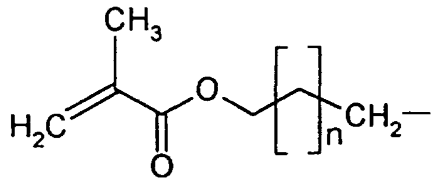

n = 0, 1, 2, 3, 4, 5, 6, 7, 8, 9, 10

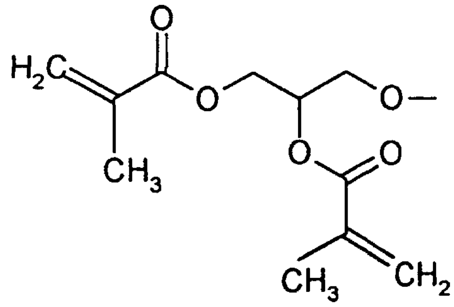

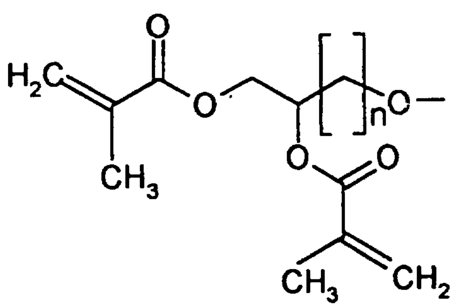

n = 1, 2, 3, 4, 5, 6, 7, 8, 9, 10

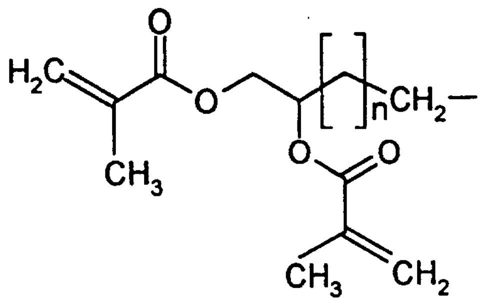

n = 0, 1, 2, 3, 4, 5, 6, 7, 8, 9, 10

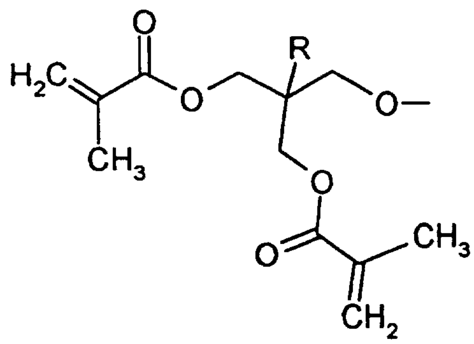

R = alkyl, aryl, alkylaryl

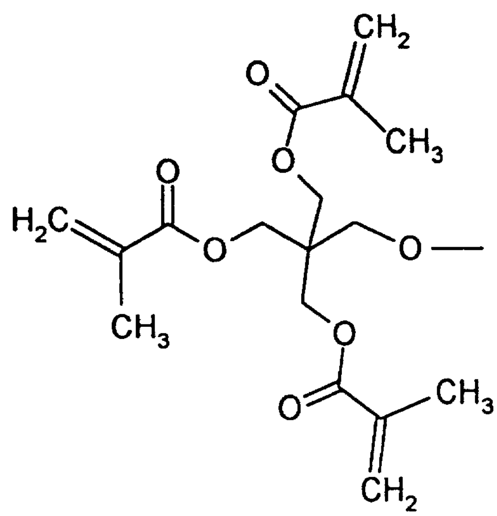

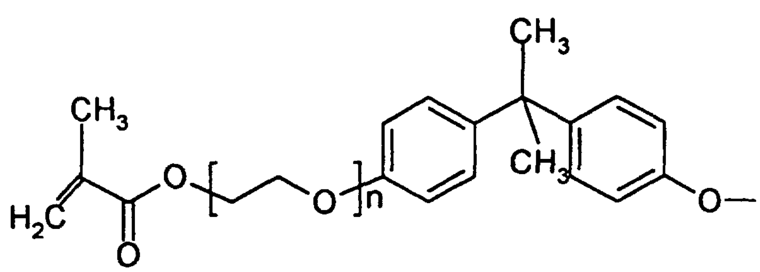

n = 1 , 2, 3, 4, 5

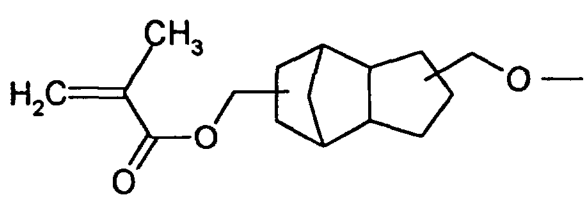

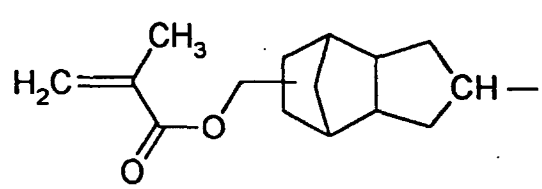

R' = alkylene, alkenylene, arylene, arylenealkylene or alkylenearylene in each case with 0 to 10 carbon atoms, the radicals being able to be interrupted by oxygen and sulphur atoms or by amino groups;

U =

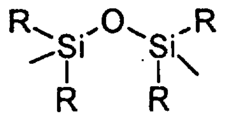

R = alkyl, aryl, cycloyalkyl

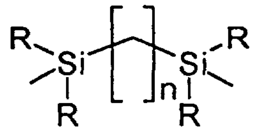

R = alkyl, aryl, cycloyalkyl
n = 1, 2, 3, 4, 5, 6, 7, 8, 9, 10

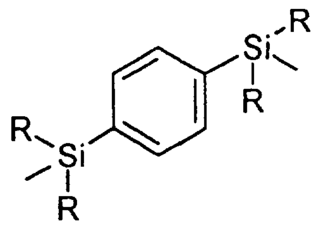

R = alkyl, aryl, cycloyalkyl

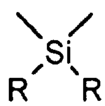

R = alkyl, aryl, cycloalkyl

D = $-(CH_2)_n-Si(CH_3)_2(OC_2H_5)$, with n = 0 to 10
$-(CH_2)_n-Si(CH_3)(OC_2H_5)_2$, with n = 0 to 10
$-(CH_2)_n-Si(OCH_3)_3$, with n = 0 to 10
$-(CH_2)_n-Si(C_2H_5)_2(OCH_3)$, with n = 0 to 10
$-(CH_2)_n-Si(C_2H_5)(OCH_3)_2$, with n = 0 to 10
$-(CH_2)_n-Si(OC_2H_5)_3$, with n = 0 to 10
$-(CH_2)_n-Si(CH_3)_2(OCH_3)$, with n = 0 to 10
$-(CH_2)_n-Si(CH_3)(OCH_3)_2$, with n = 0 to 10
$-(CH_2)_n-Si(C_2H_5)_2(OC_2H_5)$, with n = 0 to 10
$-(CH_2)_n-Si(C_2H_5)(OC_2H_5)_2$, with n = 0 to 10
$-(CH_2)_n-Si(CH_3)(C_2H_5)(OCH_3)$, with n = 0 to 10
$-(CH_2)_n-Si(CH_3)(C_2H_5)(OC_2H_5)$, with n = 0 to 10
$-(CH_2)_n-Si(CH_3)(OC_2H_5)(OCH_3)$, with n = 0 to 10
$-(CH_2)_n-Si(C_2H_5)(OC_2H_5)(OCH_3)$, with n = 0 to 10
$-(CH_2)_n-Si(OC_2H_5)_2(OCH_3)$, with n = 0 to 10
$-(CH_2)_n-Si(OC_2H_5)(OCH_3)_2$, with n = 0 to 10
$-(CH_2)_n-Si(CH_3)(OC(CH_3)=CH_2)_2$, with n = 0 to 10
$-(CH_2)_n-Si(C_2H_5)(OC(CH_3)=CH_2)_2$, with n = 0 to 10

$-(CH_2)_n-Si(CH_3)_2(OC(CH_3)=CH_2)$, with n = 0 to 10
$-(CH_2)_n-Si(C_2H_5)_2(OC(CH_3)=CH_2)$, with n = 0 to 10
$-(CH_2)_n-Si(OCH_3)(OC(CH_3)=CH_2)_2$, with n = 0 to 10
$-(CH_2)_n-Si(OC_2H_5)(OC(CH_3)=CH_2)_2$, with n = 0 to 10
$-(CH_2)_n-Si(OCH_3)_2(OC(CH_3)=CH_2)$, with n = 0 to 10
$-(CH_2)_n-Si(OC_2H_5)_2(OC(CH_3)=CH_2)$, with n = 0 to 10
$-(CH_2)_n-Si(C_6H_5)(OC_2H_5)_2$ with n = 0 to 10
$-(CH_2)_n-Si(C_6H_5)(OCH_3)_2$, with n = 0 to 10
$-(CH_2)_n-Si(C_6H_5)(OCH_3)(OC_2H_5)$, with n = 0 to 10
$-(CH_2)_n-Si(C_6H_5)(OC(CH_3)=CH_2)_2$, with n = 0 to 10.

**2.** Silanes according to claim 1, **characterized in that**, in the general formula, the radical R has the following meaning:

R' = alkylene, alkenylene, arylene, arylenealkylene or alkylenearylene in each case with 0 to 5 carbon atoms, these radicals being able to be interrupted by oxygen and sulphur atoms or by amino groups.

**3.** Process for the preparation of the silanes according to claim 1 or 2, **characterized in that** a dihydridosilicon compound is reacted in equimolar manner with an alkenyl silane compound which contains hydrolyzable groups, and the residual hydridosilyl group is reacted by renewed hydrosilylation with an at least twice-unsaturated organic compound.

**4.** Use of the silanes according to claim 1 or 2 for the preparation of silicic acid polycondensates or of silicic acid heteropolycondensates by hydrolytic condensation of one or more hydrolytically condensable compounds of silicon and optionally other elements from the group B, Al, P, Sn, Pb, the transition metals, the lanthanides and the actinides, and/or precondensates derived from the above-named compounds, optionally in the presence of a catalyst and/or a solvent, by the action of water or moisture, **characterized in that** 5 to 100 mol-% based on monomeric compounds of the hydrolytically condensable compounds are selected from silanes of the general formula I:

$$B\text{-}R'\text{-}U\text{-}D \qquad (I)$$

in which the radicals are as defined in claim 1.

**5.** Use according to claim 4, **characterized in that** compounds which can be radically or ionically polymerized are used, optionally in precondensed form, as further hydrolytically condensable compounds.

**6.** Use according to claim 4 or 5, **characterized in that** one or more compounds of the general formula VI are used, optionally in precondensed form, as further hydrolytically condensable compounds of silicon:

$$R_e(R^{10}Z')_fSiX_{4-(e+f)} \qquad (VI)$$

in which the radicals and indices have the following meaning:

X = hydrogen, halogen, hydroxy, alkoxy, acyloxy, alkylcarbonyl, alkoxycarbonyl or $NR''_2$;
R = alkyl, alkenyl, aryl, alkylaryl or arylalkyl;
R" = hydrogen, alkyl or aryl;
$R^{10}$ = alkylene or alkenylene, these radicals being able to be interrupted by oxygen or sulphur atoms or -NH groups;
Z' = halogen or an optionally substituted amino, amide, aldehyde, alkylcarbonyl, carboxy, mercapto, cyano, alkoxy, alkoxycarbonyl, sulfonic acid, phosphoric acid, acryloxy, methacryloxy, epoxy or vinyl group;
e = 0,1,2 or 3;
f = 0, 1,2 or 3, with e + f = 1,2 or 3.

**7.** Use according to one of claims 4 to 6, **characterized in that** one or more compounds of the general formula VIII are used, optionally in precondensed form, as further hydrolytically condensable compounds of silicon:

$$Y_nSiX_mR_{4-(n+m)} \quad (VIII)$$

in which the radicals X and R have the meaning given in claim 6 and the other radicals and indices have the following meaning:

Y = a substituent which contains a substituted or unsubstituted 1,4,6-trioxaspiro-[4,4]-nonane radical;
n = 1,2 or 3;
m = 1, 2 or 3, with $n + m \leqq 4$

**8.** Use according to one of claims 4 to 7, **characterized in that** one or more aluminium, titanium or zirconium compounds, soluble in the reaction medium, of the formula:

$$AlR^0 \text{ or } MX_yR_z$$

are used, optionally in precondensed form, as further hydrolytically condensable components, in which M stands for titanium or zirconium, the radicals R, $R^0$ and X are the same or different, $R^0$ represents halogen, hydroxy, alkoxy or acyloxy, y is an integer from 1 to 4, in particular 2 to 4, z stands for 0, 1, 2 or 3, preferably for 0, 1 or 2 and X and R are as defined in claim 6.

**9.** Use according to one or more of claims 4 to 8, **characterized in that** one or more initiators are added to the polycondensate, and the polycondensate cures thermally, photochemically, in covalent-nucleophilic manner or by redox-induction.

**10.** Use according to one or more of claims 4 to 8, **characterized in that** one or more radically and/or ionically polymerizable components are added to the polycondensate before polymerization.

**11.** Use of the silanes according to claim 1 for the preparation of polymerisates by radical polymerization of compounds containing one or more C=C double bonds and optionally other radically polymerizable compounds and optionally by ionic polymerization of one or more ionically polymerizable compounds, by the action of heat and/or electromagnetic radiation and/or by redox-induction and/or in covalent-nucleophilic manner, optionally in the presence of one or more initiators and/or a solvent, **characterized in that** 5 to 100 mol-% based on monomeric compounds are selected from silanes of formula I:

$$B\text{-}R'\text{-}U\text{-}D \quad (I)$$

in which the radicals are as defined in claim 1 or 2.

**12.** Use according to claim 11, **characterized in that** one or more silanes of the general formula VIII are used as cationically polymerizable compounds:

$$Y_nSiX_mR_{4-(n+m)} \quad (VIII)$$

in which the radicals and indices are as defined in claim 7.

**13.** Use according to one or more of claims 11 to 12, **characterized in that** the polymerisate is hydrolytically condensed, optionally in the presence of further, hydrolytically condensable compounds of silicon and optionally other elements from the group B, Al, Sn, Pb, the transition metals, the lanthanides and the actinides, and/or precondensates derived from the above-named compounds by the action of water or moisture, optionally in the presence of a catalyst and/or a solvent.

**14.** Use according to claim 13, **characterized in that** one or more compounds of the general formula VI are used, optionally in precondensed form, as further condensable compounds of silicon:

$$R_e(R^{10}Z')_f SiX_{4-(e+f)} \quad (VI)$$

in which the radicals and indices are as defined in claim 6.

**15.** Use according to one of claims 4 to 10, **characterized in that** one or more compounds of the general formula IX are used, optionally in precondensed form, as further condensable compounds of silicon:

$$G\{A-(Z)_d-R^{20}(R^{21})-R'-SiX_aR_b\}_c \quad (IX)$$

in which the radicals and indices have the following meaning:

X = hydrogen, halogen, hydroxy, alkoxy, acyloxy, alkylcarbonyl, alkoxycarbonyl or $NR''_2$;
R = alkyl, alkenyl, aryl, alkylaryl or arylalkyl;
R' = alkylene, alkenylene, arylene, arylenealkylene or alkylenearylene in each case with 0 to 10 carbon atoms, these radicals being able to be interrupted by oxygen and sulphur atoms or by amino groups;
R" = hydrogen, alkyl or aryl;
G = a straight-chained or branched organic radical with at least one C=C double bond and 4 to 50 carbon atoms;
A = O, S or NH for d = 1 and

Z = CO and
$R^{20}$ = alkylene, arylene or alkylenearylene in each case with 1 to 10 carbon atoms, these radicals being able to be interrupted by oxygen and sulphur atoms or by amino groups, and
$R^{21}$ = COOH;

or

A = O, S or NH for d = 1 and

Z = CO and
$R^{20}$ = alkylene, arylene or alkylenearylene in each case with 1 to 10 carbon atoms, these radicals being able to be interrupted by oxygen and sulphur atoms or by amino groups, and
$R^{21}$ = H;

or

A = O, S, NH or COO for d = 1 and

Z = CHR, with R equal to H, alkyl, aryl or alkylaryl, and
$R^{20}$ = alkylene, arylene or alkylenearylene in each case with 1 to 10 carbon atoms, these radicals being able to be interrupted by oxygen and sulphur atoms or by amino groups, and
$R^{21}$ = OH;

or

A = O, S, NH or COO for d = 0 and

$R^{20}$ = alkylene, arylene or alkylenearylene in each case with 1 to 10 carbon atoms, these radicals being able to be interrupted by oxygen and sulphur atoms or by amino groups, and
$R^{21}$ = OH;

or

A = S for d = 1 and

Z = CO and

$R^{20}$ = N and
$R^{21}$ = H;

a = 1, 2 or 3;
b = 0,1 or 2;
a+b = 3;
c = 1,2,3 or 4;
d = 0 or 1.

**16.** Use of the silanes according to claim 1 or 2 in the dental field for the preparation of filling materials, cements, temporary crown and bridge materials, facing materials, lacquers, sealers, adhesion promoters, primers and bondings.

## Revendications

**1.** Silanes hydrolysables et polymérisables de la formule générale I :

$$B\text{-}R'\text{-}U\text{-}D \quad (I)$$

dans laquelle les radicaux ont la signification suivante :

B = vinylcyclopropyle,

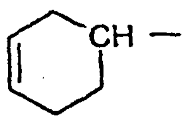

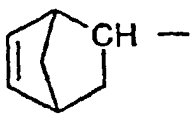

ou des radicaux de méthacrylate (y compris les radicaux d'acrylate correspondants) du groupe suivant :

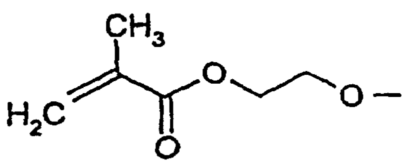

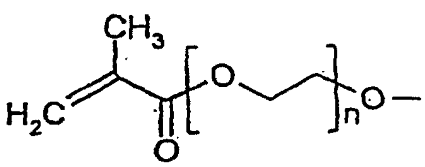

n = 2, 3, 4, 5, 6, 7,
ou des mélanges avec n moyen < 10

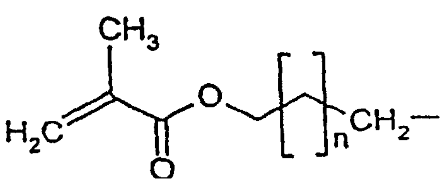

n = 0, 1, 2, 3, 4, 5, 6, 7, 8, 9, 10

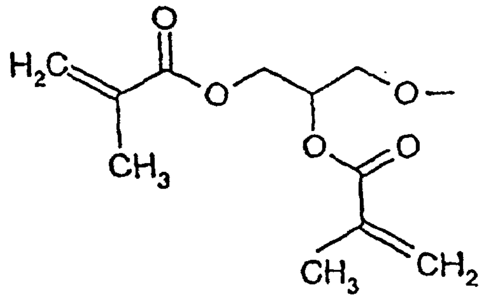

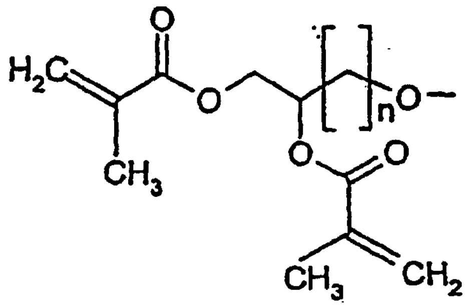

n = 1, 2, 3, 4, 5, 6, 7, 8, 9, 10

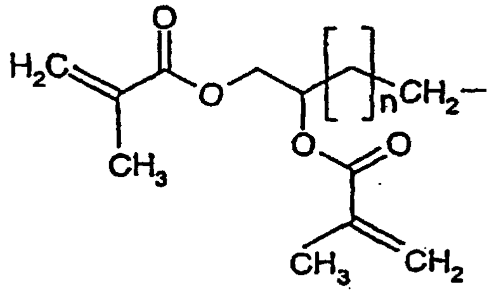

n = 0, 1, 2, 3, 4, 5, 6, 7, 8, 9, 10

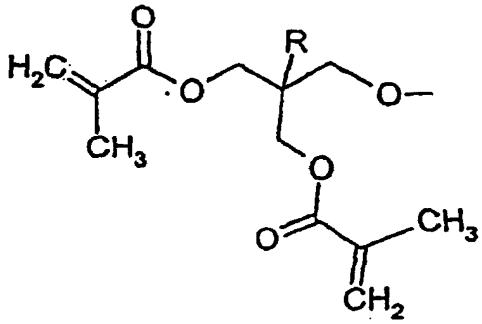

R = alkyle, aryle, alkylaryle

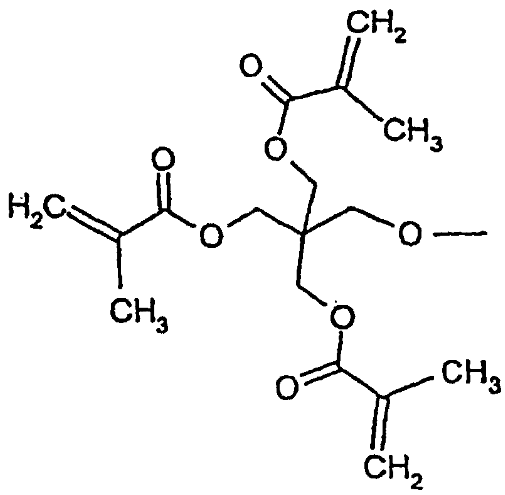

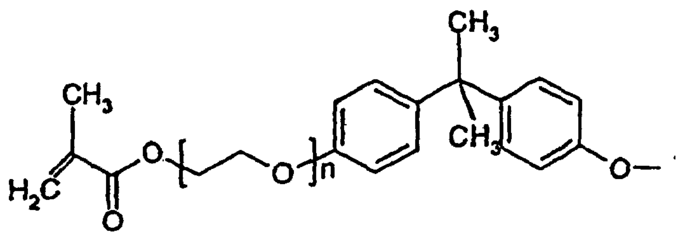

n = 1, 2, 3, 4, 5

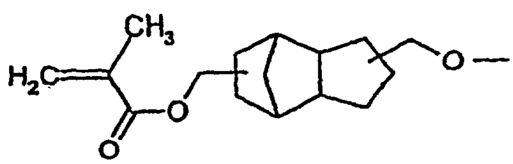 , 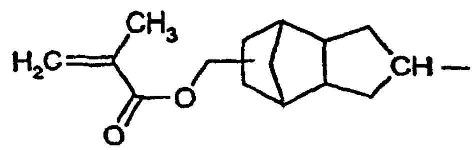 ;

R' = alkylène, alcénylène, arylène, arylènealkylène ou alkylènearylène ayant chacun 0 à 10 atomes de carbone, ces radicaux pouvant être interrompus par des atomes d'oxygène et de soufre ou par des groupes amino,

U=

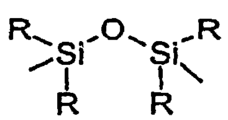

R = alkyle, aryle, cycloalkyle,

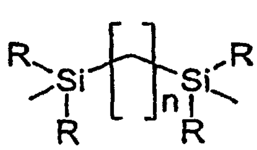

R = alkyle, aryle, cycloalkyle
n = 1,2,3,4,5,6,7,8,9,10

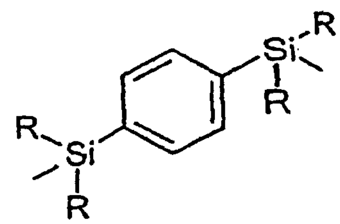

R = alkyle, aryle, cycloalkyle

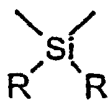

R = alkyle, aryle, cycloalkyle

D = $-(CH_2)_n-Si(CH_3)_2(OC_2H_5)$, avec n = 0 à 10
$-(CH_2)_n-Si(CH_3)(OC_2H_5)_2$, avec n = 0 à 10
$-(CH_2)_n-Si(OCH_3)_3$, avec n = 0 à 10
$-(CH_2)_n-Si(C_2H_5)_2(OCH_3)$, avec n = 0 à 10
$-(CH_2)_n-Si(C_2H_5)(OCH_3)_2$, avec n = 0 à 10
$-(CH_2)_n-Si(OC_2H_5)_3$, avec n = 0 à 10
$-(CH_2)_n-Si(CH_3)_2(OCH_3)$ avec n = 0 à 10
$-(CH_2)_n-Si(CH_3)(OCH_3)_2$, avec n = 0 à 10
$-(CH_2)_n-Si(C_2H_5)_2(OC_2H_5)$, avec n = 0 à 10
$-(CH_2)_n-Si(C_2H_5)(OC_2H_5)_2$, avec n = 0 à 10
$-(CH_2)_n-Si(CH_3)(C_2H_5)(OCH_3)$, avec n = 0 à 10
$-(CH_2)_n-Si(CH_3)(C_2H_5)(OC_2H_5)$, avec n = 0 à 10
$-(CH_2)_n-Si(CH_3)(OC_2H_5)(OCH_3)$, avec n = 0 à 10
$-(CH_2)_n-Si(C_2H_5)(OC_2H_5)(OCH_3)$, avec n = 0 à 10
$-(CH_2)_n-Si(OC_2H_5)_2(OCH_3)$, avec n = 0 à 10
$-(CH_2)_n-Si(OC_2H_5)(OCH_3)_2$, avec n = 0 à 10
$-(CH_2)_n-Si(CH_3)(OC(CH_3)=CH_2)_2$, avec n = 0 à 10
$-(CH_2)_n-Si(C_2H_5)(OC(CH_3)=CH_2)_2$, avec n = 0 à 10
$-(CH_2)_n-Si(CH_3)_2(OC(CH_3)=CH_2)$, avec n = 0 à 10
$-(CH_2)_n-Si(C_2H_5)_2(OC(CH_3)=CH_2)$, avec n = 0 à 10
$-(CH_2)_n-Si(OCH_3)(OC(CH_3)=CH_2)_2$, avec n = 0 à 10
$-(CH_2)_n-Si(OC_2H_5)(OC(CH_3)=CH_2)_2$, avec n = 0 à 10
$-(CH_2)_n-Si(OCH_3)(OC(CH_3)=CH_2)$, avec n = 0 à 10
$-(CH_2)_n-Si(OC_2H_5)_2(OC(CH_3)=CH_2)$, avec n = 0 à 10
$-(CH_2)_n-Si(C_6H_5)(OC_2H_5)_2$, avec n = 0 à 10
$-(CH_2)_n-Si(C_6H_5)(OCH_3)_2$, avec n = 0 à 10
$-(CH_2)_n-Si(C_6H_5)(OCH_3)(OC_2H_5)$, avec n = 0 à 10
$-(CH_2)_n-Si(C_6H_5)(OC(CH_3)=CH_2)_2$, avec n = 0 à 10

2. Silanes suivant la revendication 1, **caractérisés en ce que**, dans la formule générale, le radical R' a la signification suivante :

R' = alkylène, alcénylène, arylène, arylènealkylène ou alkylènearylène comportant chacun 0 à 5 atomes de carbone, ces radicaux pouvant être interrompus par des atomes d'oxygène et de soufre ou par des groupes amino.

3. Procédé de préparation des silanes suivant l'une des revendications 1 et 2, **caractérisé en ce qu'**on fait réagir de manière équimolaire un composé de dihydridosilicium avec un composé d'alcénylsilane, qui contient des groupes hydrolysables, et **en ce qu'**on fait réagir le groupe hydridosilyle subsistant, par une nouvelle hydrosilylation, avec un composé organique au moins deux fois insaturé.

**4.** Utilisation de silanes suivant l'une des revendications 1 et 2, pour la préparation de polycondensats d'acide silicique ou d'hétéropolycondensats d'acide silicique par condensation hydrolytique d'un ou de plusieurs composés du silicium condensables par hydrolyse et éventuellement d'autres éléments du groupe de B, Al, P, Sn, Pb, des métaux de transition, des lanthanides et des actinides, et/ou des précondensats dérivés des composés précités, éventuellement en présence d'un catalyseur et/ou d'un solvant, par action d'eau ou d'humidité, **caractérisée en ce qu'**on sélectionne 5 à 100 moles %, sur base des composés monomères des composés condensables par hydrolyse, dans des silanes de la formule générale I :

$$B\text{-}R'\text{-}U\text{-}D \quad (I)$$

dans laquelle les radicaux sont définis comme dans la revendication 1.

**5.** Utilisation suivant la revendication 4, **caractérisée en ce que**, comme composés condensables par hydrolyse, on met en oeuvre des composés, éventuellement sous forme précondensée, qui sont polymérisables par voie radicalaire ou ionique.

**6.** Utilisation suivant l'une des revendications 4 et 5, **caractérisée en ce que**, comme autres composés du silicium condensables par hydrolyse, on met en oeuvre un ou plusieurs composés de la formule générale VI, éventuellement sous forme précondensée :

$$R_e(R^{10}Z')_fSiX_{4-(e+f)} \quad (VI)$$

dans laquelle les radicaux et indices ont la signification suivante :

X = hydrogène, halogène, hydroxy, alcoxy, acyloxy, alkyl-carbonyle, alcoxycarbonyle ou $NR''_2$,
R = alkyle, alcényle, aryle, alkylaryle ou arylalkyle,
R" = hydrogène, alkyle ou aryle,
$R^{10}$ = alkylène ou alcénylène, ces radicaux pouvant être interrompus par des atomes d'oxygène ou de soufre ou des groupes NH,
Z' = halogène ou un groupe amino, amide, aldéhyde, alkyl-carbonyle, carboxy, mercapto, cyano, alcoxy, alcoxy-carbonyle, acide sulfonique, acide phosphorique, acryloxy, méthacryloxy, époxy ou vinyle, éventuellement substitué,
e= 0, 1, 2 ou 3,
f= 0, 1, 2 ou 3, avec e + f = 1, 2 ou 3.

**7.** Utilisation suivant l'une des revendications 4 à 6, **caractérisée en ce que**, comme autres composés du silicium condensables par hydrolyse, on met en oeuvre un ou plusieurs composés de la formule générale VIII, éventuellement sous forme précondensée :

$$Y_n\ Si\ X_m\ R_{4-(n+m)} \quad (VIII)$$

dans laquelle X et R ont la signification indiquée dans la revendication 6 et les autres radicaux et indices ont la signification suivante :

Y = un substituant qui contient un radical de 1,4,6-trioxaspiro-[4,4]-nonane substitué ou non substitué,
n = 1, 2 ou 3,
m = 1, 2 ou 3, avec $n + m \leqq 4$.

**8.** Utilisation suivant l'une des revendications 4 à 7, **caractérisée en ce que**, comme autres composants condensables par hydrolyse, on met en oeuvre un ou plusieurs composés d'aluminium, de titane ou de zirconium solubles dans le milieu réactionnel, éventuellement sous forme précondensée, répondant aux formules :

$$AlR^0 \text{ ou } M\ X_y\ R_z$$

dans lesquelles M représente du titane ou du zirconium, les radicaux R, $R^0$ et X sont identiques ou différents, $R^0$ représente un halogène, de l'hydroxy, de l'alcoxy ou de l'acyloxy, y est un nombre entier de 1 à 4, en particulier de 2 à 4, z vaut 0, 1, 2 ou 3, de préférence 0, 1 ou 2, et X et R sont définis comme dans la revendication 6.

**9.** Utilisation suivant une ou plusieurs des revendications 4 à 8, **caractérisée en ce qu'**on ajoute au polycondensat un ou plusieurs amorceurs et **en ce que** le polycondensat durcit par voie thermique, photochimique, covalente-nucléophile ou induite par oxydoréduction.

**10.** Utilisation suivant une ou plusieurs des revendications 4 à 8, **caractérisée en ce qu'**on ajoute au polycondensat, avant la polymérisation, un ou plusieurs composants polymérisables par voie radicalaire et/ou ionique.

**11.** Utilisation des silanes suivant la revendication 1, pour la préparation de polymères par polymérisation radicalaire d'un ou de plusieurs composés contenant des doubles liaisons C=C et éventuellement d'autres composés polymérisables par voie radicalaire et éventuellement par polymérisation ionique d'un ou de plusieurs composés polymérisables par voie ionique, par action de chaleur et/ou d'un rayonnement électromagnétique et/ou de manière induite par oxydoréduction et/ou par voie covalente-nucléophile, éventuellement en présence d'un ou de plusieurs amorceurs et/ou d'un solvant, **caractérisée en ce qu'**on sélectionne 5 à 100 moles %, sur base des composés monomères, dans des silanes de la formule I:

$$B\text{-}R'\text{-}U\text{-}D \qquad (I)$$

dans laquelle les radicaux sont définis comme dans l'une des revendications 1 et 2.

**12.** Utilisation suivant la revendication 11, **caractérisée en ce que**, comme composés polymérisables par voie cationique, on met en oeuvre un ou plusieurs silanes de la formule générale VIII :

$$Y_n\ Si\ X_m\ R_{4-(n+m)} \qquad (VIII)$$

dans laquelle les radicaux et indices sont définis comme dans la revendication 7.

**13.** Utilisation suivant une ou plusieurs des revendications 11 et 12, **caractérisée en ce qu'**on condense par hydrolyse le polymère, éventuellement en présence d'autres composés du silicium condensables par hydrolyse et éventuellement d'autres éléments du groupe de B, Al, Sn, Pb, des métaux de transition, des lanthanides et des actinides, et/ou de précondensats dérivés des composés cités ci-dessus, par action d'eau ou d'humidité, éventuellement en présence d'un catalyseur et/ou d'un solvant.

**14.** Utilisation suivant la revendication 13, **caractérisée en ce que**, comme autres composés du silicium condensables, on met en oeuvre un ou plusieurs composés de la formule générale VI, éventuellement sous forme précondensée :

$$R_e(R^{10}Z')_fSiX_{4-(e+f)} \qquad (VI)$$

dans laquelle les radicaux et indices sont définis comme dans la revendication 6.

**15.** Utilisation suivant l'une des revendications 4 à 10, **caractérisée en ce que**, comme autres composés du silicium condensables, on met en oeuvre un ou plusieurs composés de la formule générale IX, éventuellement sous forme précondensée :

$$G\{A\text{-}(Z)_d\text{-}R^{20}(R^{21})\text{-}R'\text{-}SiX_aR_b\}_c \qquad (IX)$$

dans laquelle les radicaux et indices ont la signification suivante :

X = hydrogène, halogène, hydroxy, alcoxy, acyloxy, alkyl-carbonyle, alcoxycarbonyle ou $NR''_2$,

R = alkyle, alcényle, aryle, alkylaryle ou arylalkyle,
R' = alkylène, alcénylène, arylène, arylènealkylène ou alkylènearylène comportant chacun 0 à 10 atomes de carbone, ces radicaux pouvant être interrompus par des atomes d'oxygène et de soufre ou par des groupes amino,
R"= hydrogène, alkyle ou aryle,
G = un radical organique linéaire ou ramifié comportant au moins une double liaison C=C et 4 à 50 atomes de carbone,
A = O, S ou NH, pour d = 1, et

Z = CO, et
$R^{20}$= alkylène, arylène ou alkylènearylène comportant chacun 1 à 10 atomes de carbone, ces radicaux pouvant être interrompus par des atomes d'oxygène et de soufre ou par des groupes amino, et
$R^{21}$= COOH,

ou

A = O, S ou NH, pour d = 1, et

Z= CO, et
$R^{20}$ = alkylène, arylène ou alkylènearylène comportant chacun 1 à 10 atomes de carbone, ces radicaux pouvant être interrompus par des atomes d'oxygène et de soufre ou par des groupes amino, et
$R^{21}$ = H,

ou

A = O, S, NH ou COO, pour d = 1, et

Z = CHR, où R est égal à H, alkyle, aryle ou alkylaryle, et
$R^{20}$ = alkylène, arylène ou alkylènearylène comportant chacun 1 à 10 atomes de carbone, ces radicaux pouvant être interrompus par des atomes d'oxygène et de soufre ou par des groupes amino, et
$R^{21}$ = OH,

ou

A = O, S, NH ou COO, pour d = 0, et

$R^{20}$ = alkylène, arylène ou alkylènearylène comportant chacun 1 à 10 atomes de carbone, ces radicaux pouvant être interrompus par des atomes d'oxygène et de soufre ou par des groupes amino, et
$R^{21}$ = OH,

ou

A = S, pour d = 1, et

Z= CO, et
$R^{20}$ = N, et
$R^{21}$ = H,

a = 1, 2 ou 3,
b = 0, 1 ou 2,
a + b = 3,
c =1, 2, 3 ou 4,
d = 0 ou 1.

**16.** Utilisation des silanes suivant l'une des revendications 1 et 2, dans le domaine dentaire, pour la préparation de matières de remplissage, de ciments, de matières de couronnes et de ponts provisoires, de matières de revêtement, de laques, de matières de scellement, de matières adhérisantes, de matières primaires et de matières liantes.